# EUROPEAN PATENT SPECIFICATION

(11) **EP 3 596 222 B1**
(45) Date of publication and mention of the grant of the patent: **12.04.2023**
(21) Application number: 18768395.8
(22) Date of filing: 16.03.2018
(51) Int. Cl.: C12N 15/86, C12N 15/113, C12N 15/09

(54) **ADENO-ASSOCIATED VIRUS VECTOR DELIVERY OF MUSCLE SPECIFIC MICRO-DYSTROPHIN TO TREAT MUSCULAR DYSTROPHY**
ADENO-ASSOZIIERTE VIRUSVEKTORVERABREICHUNG VON MUSKELSPEZIFISCHEM MIKRODYSTROPHIN ZUR BEHANDLUNG VON MUSKELDYSTROPHIE
ADMINISTRATION PAR VECTEUR À VIRUS ADÉNO-ASSOCIÉ DE MICRO-DYSTROPHINE SPÉCIFIQUE DU MUSCLE POUR TRAITER LA DYSTROPHIE MUSCULAIRE

(30) Priority: 17.03.2017 US 201762473148 P
(43) Date of publication of application: 22.01.2020
(73) Proprietor: Research Institute at Nationwide Children's Hospital, Columbus, Ohio 43205 (US)
(72) Inventor: RODINO-KLAPAC, Louise, Grove City, OH 43123 (US); MENDELL, Jerry, R., Columbus, OH 43235 (US)
(74) Representative: D Young & Co LLP
(86) International application number: PCT/US2018/022881
(87) International publication number: WO 2018/170408

(56) References cited:
- WO-A1-2015/197232
- WO-A1-2016/177911
- WO-A1-2017/181014
- US-A1- 2008 044 393
- MAJA Z SALVA ET AL: "Design of Tissue-specific Regulatory Cassettes for High-level rAAV-mediated Expression in Skeletal and Cardiac Muscle", MOLECULAR THERAPY, vol. 15, no. 2, 1 February 2007 (2007-02-01), pages 320-329, XP55078517, ISSN: 1525-0016, DOI: 10.1038/sj.mt.6300027
- HELLER ET AL: "379. MicroRNA-29 and Micro-Dystrophin Combinatorial Therapy Suppresses Fibrosis and Restores Function to mdx/utrn+/- Mice", MOLECULAR THERAPY, vol. 24, no. 1, 14 December 2016 (2016-12-14), page S151, XP055433385, DOI: 10.1016/S1525-0016(16)33188-4
- Paul T. Martin ET AL: "Translational Studies of GALGT2 Gene therapy for Duchenne Muscular Dystropy", , 1 January 2013 (2013-01-01), XP55535911, Retrieved from the Internet: URL:https://apps.dtic.mil/dtic/tr/fulltext /u2/a613577.pdf [retrieved on 2018-12-18]
- HARPER SCOTT Q ET AL: "Modular flexibility of dystrophin: Implications for gene therapy of Duchenne muscular dystrophy", NATURE MEDICINE, NATURE PUB. CO, NEW YORK, vol. 8, no. 3, 1 March 2002 (2002-03-01), pages 253-261, XP002604007, ISSN: 1078-8956
- GUAN XUAN ET AL: "Gene therapy in monogenic congenital myopathies", METHODS, vol. 99, 14 October 2015 (2015-10-14), pages 91-98, XP029499274, ISSN: 1046-2023, DOI: 10.1016/J.YMETH.2015.10.004
- JAYNES, JB et al.: "Transcriptional Regulation ot the Muscle Creatine Kinase Gene and Regulated Expression in Transfected Mouse Myoblasts", Molecular and Cell Biology, vol. 6, no. 8, August 1986 (1986-08), pages 2855-2864, XP055541442,
- LEDERFEIN, D et al.: "A 71-Kilodalton Protein is a Major Product of the Duchenne Muscular Dystrophy Gene in Brain and Other Nonmuscle Tissues", PNAS, vol. 89, no. 12, 15 June 1992 (1992-06-15) , pages 5346-5350, XP055541447,

## Description

### FIELD OF INVENTION

The invention provides adeno-associated virus (AAV) vectors expressing a miniaturized human micro-dystrophin gene and uses of these vectors in methods to express micro-dystrophin in skeletal muscles including diaphragm and cardiac muscle and to protect muscle fibers from injury, increase muscle strength and reduce and/or prevent fibrosis in subjects suffering from muscular dystrophy.

### BACKGROUND

The importance of muscle mass and strength for daily activities, such as locomotion and breathing, and for whole body metabolism is unequivocal. Deficits in muscle function produce muscular dystrophies (MDs) that are characterized by muscle weakness and wasting and have serious impacts on quality of life. The most well-characterized MDs result from mutations in genes encoding members of the dystrophin-associated protein complex (DAPC). These MDs result from membrane fragility associated with the loss of sarcolemmal-cytoskeleton tethering by the DAPC. Duchenne Muscular Dystrophy (DMD) is one of the most devastating muscle disease affecting 1 in 5000 newborn males.

DMD is caused by mutations in the DMD gene leading to reductions in mRNA and the absence of dystrophin, a 427 kD sarcolemmal protein associated with the dystrophin-associated protein complex (DAPC) (Hoffman et al., Cell 51(6):919-28, 1987). The DAPC is composed of multiple proteins at the muscle sarcolemma that form a structural link between the extra-cellular matrix (ECM) and the cytoskeleton via dystrophin, an actin binding protein, and alpha-dystroglycan, a laminin-binding protein. These structural links act to stabilize the muscle cell membrane during contraction and protect against contraction-induced damage. With dystrophin loss, membrane fragility results in sarcolemmal tears and an influx of calcium, triggering calcium-activated proteases and segmental fiber necrosis (Straub et al., Curr Opin. Neurol. 10(2): 168-75, 1997). This uncontrolled cycle of muscle degeneration and regeneration ultimately exhausts the muscle stem cell population (Sacco et al., Cell, 2010. 143(7): p. 1059-71; Wallace et al., Annu Rev Physiol, 2009. 71: p. 37-57), resulting in progressive muscle weakness, endomysial inflammation, and fibrotic scarring.

Without membrane stabilization from dystrophin or a micro-dystrophin, DMD will manifest uncontrolled cycles of tissue injury and repair ultimately replace lost muscle fibers with fibrotic scar tissue through connective tissue proliferation. Fibrosis is characterized by the excessive deposits of ECM matrix proteins, including collagen and elastin. ECM proteins are primarily produced from cytokines such as TGFβ that is released by activated fibroblasts responding to stress and inflammation. Although the primary pathological feature of DMD is myofiber degeneration and necrosis, fibrosis as a pathological consequence has equal repercussions. The overproduction of fibrotic tissue restricts muscle regeneration and contributes to progressive muscle weakness in the DMD patient. In one study, the presence of fibrosis on initial DMD muscle biopsies was highly correlated with poor motor outcome at a 10-year follow-up (Desguerre et al., JNeuropatholExp Neurol, 2009. 68(7): p. 762-7). These results point to fibrosis as a major contributor to DMD muscle dysfunction and highlight the need for early intervention prior to overt fibrosis.

Adeno-associated virus (AAV) is a replication-deficient parvovirus, the single-stranded DNA genome of which is about 4.7 kb in length including 145 nucleotide inverted terminal repeat (ITRs). There are multiple serotypes of AAV. The nucleotide sequences of the genomes of the AAV serotypes are known. For example, the nucleotide sequence of the AAV serotype 2 (AAV2) genome is presented in Srivastava et al., J Virol, 45: 555-564 (1983) as corrected by Ruffing et al., J Gen Virol, 75: 3385-3392 (1994). As other examples, the complete genome of AAV-1 is provided in GenBank Accession No. NC_002077; the complete genome of AAV-3 is provided in GenBank Accession No. NC_1829; the complete genome of AAV-4 is provided in GenBank Accession No. NC_001829; the AAV-5 genome is provided in GenBank Accession No. AF085716; the complete genome of AAV-6 is provided in GenBank Accession No. NC_00 1862; at least portions of AAV-7 and AAV-8 genomes are provided in GenBank Accession Nos. AX753246 and AX753249, respectively (see also U.S. Patent Nos. 7,282,199 and 7,790,449 relating to AAV-8); the AAV-9 genome is provided in Gao et al., J. Virol., 78: 6381-6388 (2004); the AAV-10 genome is provided *in* Mol. Ther., 13(1): 67-76 (2006); and the AAV-11 genome is provided in Virology, 330(2): 375-383 (2004). Cloning of the AAVrh.74 serotype is described in Rodino-Klapac., et al. Journal of translational medicine 5, 45 (2007). Cis-acting sequences directing viral DNA replication (rep), encapsidation/packaging and host cell chromosome integration are contained within the ITRs. Three AAV promoters (named p5, p19, and p40 for their relative map locations) drive the expression of the two AAV internal open reading frames encoding rep and cap genes. The two rep promoters (p5 and p19), coupled with the differential splicing of the single AAV intron (e.g., at AAV2 nucleotides 2107 and 2227), result in the production of four rep proteins (rep 78, rep 68, rep 52, and rep 40) from the rep gene. Rep proteins possess multiple enzymatic properties that are ultimately responsible for replicating the viral genome. The cap gene is expressed from the p40 promoter and it encodes the three capsid proteins VP1, VP2, and VP3. Alternative splicing and non-consensus translational start sites are responsible for the production of the three related capsid proteins. A single consensus polyadenylation site is located at map position 95 of the AAV genome. The life cycle and genetics of AAV are reviewed in Muzyczka, Current Topics in Microbiology and Immunology, 158: 97-129 (1992).

AAV possesses unique features that make it attractive as a vector for delivering foreign DNA to cells, for example, in gene therapy. AAV infection of cells in culture is noncytopathic, and natural infection of humans and other animals is silent and asymptomatic. Moreover, AAV infects many mammalian cells allowing the possibility of targeting many different tissues *in vivo.* Moreover, AAV transduces slowly dividing and non-dividing cells, and can persist essentially for the lifetime of those cells as a transcriptionally active nuclear episome (extrachromosomal element). The AAV proviral genome is infectious as cloned DNA in plasmids which makes construction of recombinant genomes feasible. Furthermore, because the signals directing AAV replication, genome encapsidation and integration are contained within the ITRs of the AAV genome, some or all of the internal approximately 4.3 kb of the genome (encoding replication and structural capsid proteins, rep-cap) may be replaced with foreign DNA such as a gene cassette containing a promoter, a DNA of interest and a polyadenylation signal. The rep and cap proteins may be provided *in trans.* Another significant feature of AAV is that it is an extremely stable and hearty virus. It easily withstands the conditions used to inactivate adenovirus (56°C to 65°C for several hours), making cold preservation of AAV less critical. AAV may even be lyophilized. Finally, AAV-infected cells are not resistant to superinfection.

Multiple studies have demonstrated long-term (> 1.5 years) recombinant AAV-mediated protein expression in muscle. See, Clark et al., Hum Gene Ther, 8: 659-669 (1997); Kessler et al., Proc Nat. Acad Sc. USA, 93: 14082-14087 (1996); and Xiao et al., J Virol, 70: 8098-8108 (1996). See also, Chao et al., Mol Ther, 2:619-623 (2000) and Chao et al., Mol Ther, 4:217-222 (2001). Moreover, because muscle is highly vascularized, recombinant AAV transduction has resulted in the appearance of transgene products in the systemic circulation following intramuscular injection as described in Herzog et al., Proc Natl Acad Sci USA, 94: 5804-5809 (1997) and Murphy et al., Proc Natl Acad Sci USA, 94: 13921-13926 (1997). Moreover, Lewis et al., J Virol, 76: 8769-8775 (2002) demonstrated that skeletal myofibers possess the necessary cellular factors for correct antibody glycosylation, folding, and secretion, indicating that muscle is capable of stable expression of secreted protein therapeutics.

Functional improvement in patients suffering from DMD and other muscular dystrophies requires gene restoration at an early stage of disease. There is a need for treatments that increase muscle strength and protect against muscle injury in patients suffering from DMD.

### SUMMARY OF INVENTION

The present invention is directed to AAV vectors expressing the micro-dystrophin gene to skeletal muscles including diaphragm and cardiac muscle to protect muscle fibers from injury, increase muscle strength and reduce and/or prevent fibrosis.

The present invention provides a recombinant AAVrh.74 vector comprising an MHCK7 muscle-specific promoter/enhancer operably linked to the nucleotide sequence of SEQ ID NO: 1, wherein the recombinant AAVrh.74 vector comprises a 5' AAV2 inverted terminal repeat (ITR), the MHCK7 muscle-specific promoter/enhancer, an SV40 intron, the nucleotide sequence of SEQ ID NO: 1, a synthetic polyadenylation (PolyA) signal and a 3' AAV2 ITR.

Described herein are therapies and approaches for increasing muscular force and/or increasing muscle mass using gene therapy vectors to deliver micro-dystrophin to address the gene defect observed in DMD. As shown in Example 2, treatment with micro-dystrophin gene therapy resulted in a greater muscle force *in vivo.* Furthermore, delivery of micro-dystrophin gene therapy intramuscularly and systemically showed delivery of dystrophin to the muscles in mice models *in vivo.*

The micro-dystrophin protein provides stability to the muscle membrane during muscle contraction, e.g. micro-dystrophin acts as a shock absorber during muscle contraction.

In one embodiment, the rAAV vector is a non-replicating, recombinant adeno-associated virus (AAV) termed rAAVrh74.MHCK7.micro-dystrophin. This vector genome contains minimal elements required for gene expression, including AAV2 inverted terminal repeats (ITR), the micro-dystrophin, SV40 intron (SD/SA), and synthetic polyadenylation (Poly A) signal, all under the control of the MHCK7 promoter/enhancer. The schematic of the vector genome and expression cassette is shown. The AAVrh74 serotype can be employed to achieve efficient gene transfer in skeletal and cardiac muscle following IV administration.

The term "stringent" is used to refer to conditions that are commonly understood in the art as stringent. Hybridization stringency is principally determined by temperature, ionic strength, and the concentration of denaturing agents such as formamide. Examples of stringent conditions for hybridization and washing are 0.015 M sodium chloride, 0.0015 M sodium citrate at 65-68°C or 0.015 M sodium chloride, 0.0015M sodium citrate, and 50% formamide at 42°C. See Sambrook et al., Molecular Cloning: A Laboratory Manual, 2nd Ed., Cold Spring Harbor Laboratory, (Cold Spring Harbor, N.Y. 1989). More stringent conditions (such as higher temperature, lower ionic strength, higher formamide, or other denaturing agent) may also be used, however, the rate of hybridization will be affected. In instances wherein hybridization of deoxyoligonucleotides is concerned, additional exemplary stringent hybridization conditions include washing in 6x SSC 0.05% sodium pyrophosphate at 37°C (for 14-base oligos), 48°C (for 17-base oligos), 55°C (for 20-base oligos), and 60°C (for 23-base oligos).

Other agents may be included in the hybridization and washing buffers for the purpose of reducing non-specific and/or background hybridization. Examples are 0.1% bovine serum albumin, 0.1% polyvinyl-pyrrolidone, 0.1% sodium pyrophosphate, 0.1% sodium dodecylsulfate, NaDodSO4, (SDS), ficoll, Denhardt's solution, sonicated salmon sperm DNA (or other non-complementary DNA), and dextran sulfate, although other suitable agents can also be used. The concentration and types of these additives can be changed without substantially affecting the stringency of the hybridization conditions. Hybridization experiments are usually carried out at pH 6.8-7.4, however, at typical ionic strength conditions, the rate of hybridization is nearly independent of pH. See Anderson et al., Nucleic Acid Hybridisation: A Practical Approach, Ch. 4, IRL Press Limited (Oxford, England). Hybridization conditions can be adjusted by one skilled in the art in order to accommodate these variables and allow DNAs of different sequence relatedness to form hybrids.

The term "muscle specific control element" refers to a nucleotide sequence that regulates expression of a coding sequence that is specific for expression in muscle tissue. These control elements include enhancers and promoters. Described herein are constructs comprising the muscle specific control elements MCKH7 promoter, the MCK promoter and the MCK enhancer.

The term "operably linked" refers to the positioning of the regulatory element nucleotide sequence, e.g. promoter nucleotide sequence, to confer expression of said nucleotide sequence by said regulatory element.

The recombinant AAV vectors of the present invention comprises an MHCK7 muscle-specific promoter/enhancer operably linked to the nucleotide sequence of SEQ ID NO: 1. Other muscle specific control elements include a human skeletal actin gene element, cardiac actin gene element, myocyte-specific enhancer binding factor (MEF), muscle creatine kinase (MCK), truncated MCK (tMCK), myosin heavy chain (MHC), hybrid α-myosin heavy chain enhancer-/MCK enhancer-promoter (MHCK7), C5-12, murine creatine kinase enhancer element, skeletal fast-twitch troponin c gene element, slow-twitch cardiac troponin c gene element, the slow-twitch troponin i gene element, hypoxia-inducible nuclear factors, steroid-inducible element or glucocorticoid response element (GRE).

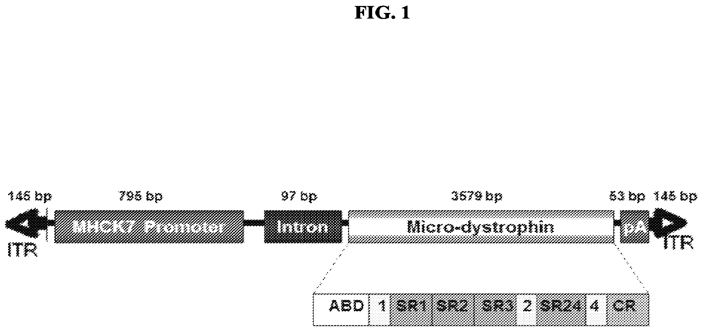

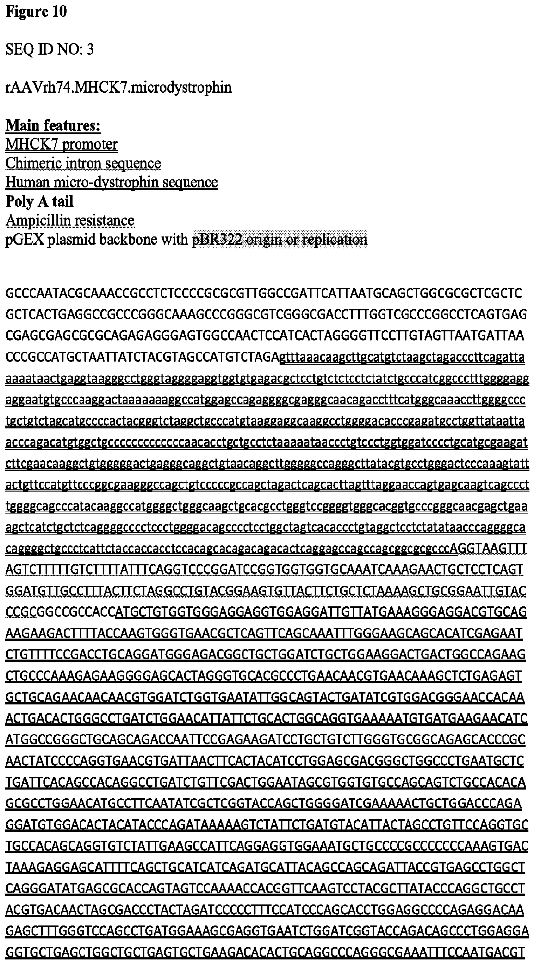

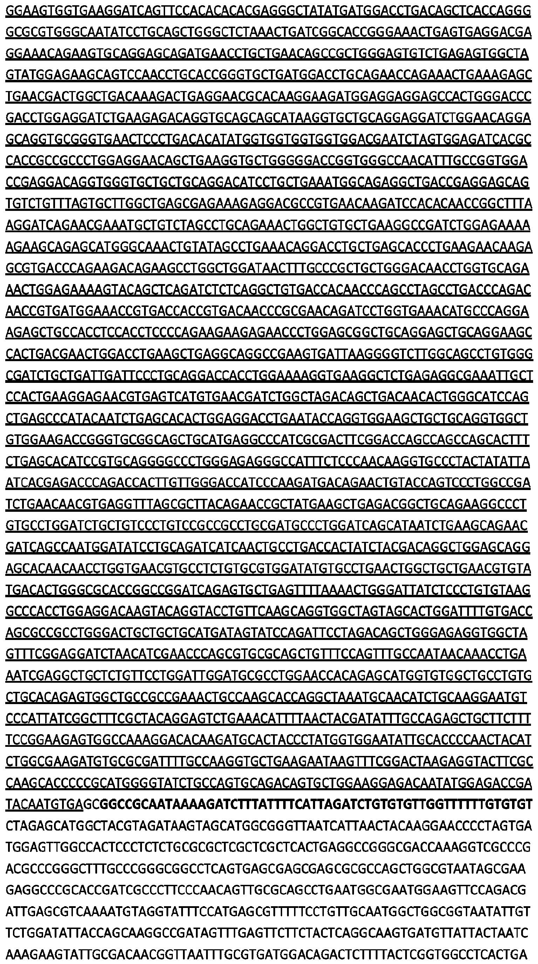

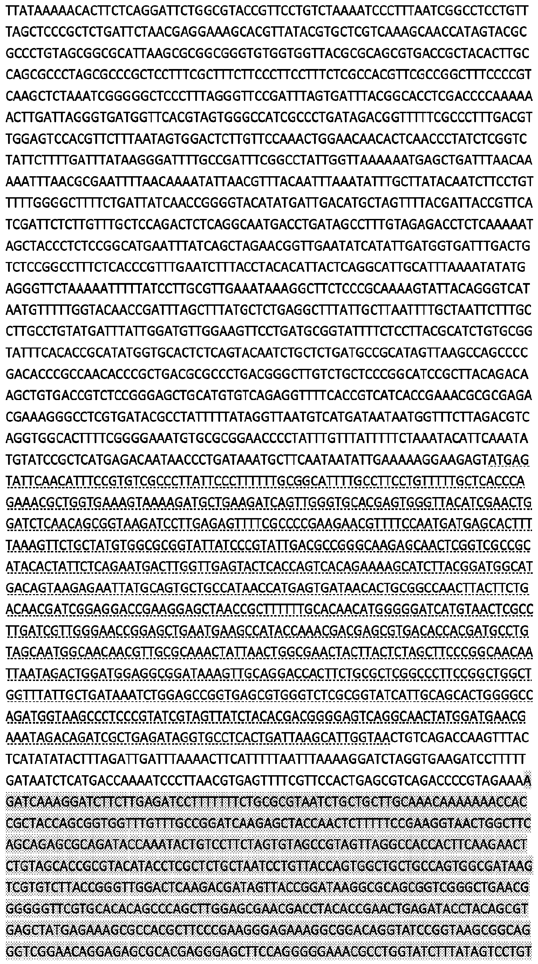

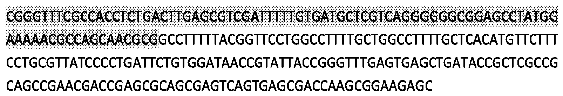

For example, a muscle specific control element is the MHCK7 promoter nucleotide sequence SEQ ID NO: 2 or a muscle specific control element is MCK nucleotide sequence SEQ ID NO: 4. In addition, in any of the rAAV vectors of the invention, the muscle specific control element nucleotide sequence is operably linked to the nucleotide sequence encoding the micro-dystrophin protein. For example, the MHCK7 promoter nucleotide sequence (SEQ ID NO: 2) is operably linked to the human micro-dystrophin coding sequence (SEQ ID NO: 1) as set out in the construct provided in Figure 1 or Figure 10 (SEQ ID NO: 3). In another aspect, the invention provides for a rAAV vector of the invention comprising the nucleotide sequence of SEQ ID NO: 1 and SEQ ID NO: 2.

In a further aspect, the invention provides for a rAAV vector of the invention comprising the nucleotide sequence of SEQ ID NO: 3. For example, the rAAVrh74.MHCK7.microdystrophin vector comprises the nucleotide sequence of SEQ ID NO: 3 and shown in Figure 10. This rAAV vector comprises the MHCK7 promoter, a chimeric intron sequence, the coding sequence for the human micro-dystrophin gene, poly A, ampicillin resistance and the pGEX plasmid backbone with pBR322 origin or replication.

The invention provides for a recombinant AAV vector of the invention comprising the human micro-dystrophin nucleotide sequence of SEQ ID NO: 1 and the MHCK7 promoter nucleotide sequence of SEQ ID NO: 3. This rAAV vector is the AAV serotype AAVrh.74.

The invention also provides for a recombinant AAV vector of the invention comprising the pAAV.MHCK7.micro-dystrophin construct nucleotide sequence of SEQ ID NO: 3. This rAAV vector is the AAV serotype AAVrh.74.

The invention also provides for pharmaceutical compositions (or sometimes referred to herein as simply "compositions") comprising any of the rAAV vectors of the invention.

Described herein are methods of producing a rAAV vector particle comprising culturing a cell that has been transfected with any rAAV vector of the invention and recovering rAAV particles from the supernatant of the transfected cells. The invention also provides for viral particles comprising any of the recombinant AAV vectors of the invention.

The invention provides for a recombinant AAV vector of the invention for use in methods of treating muscular dystrophy comprising administering a therapeutically effective amount of any of the recombinant AAV vectors of the invention expressing human micro-dystrophin.

The invention provides for a recombinant AAV vector of the invention for use in methods of treating muscular dystrophy comprising administering a therapeutically effective amount of a recombinant AAV vector of the invention comprising the human micro-dystrophin nucleotide sequence of SEQ ID NO: 1 and the MHCK7 promoter nucleotide sequence of SEQ ID NO: 2.

The invention also provides for a recombinant AAV vector of the invention for use in methods of treating muscular dystrophy comprising administering a therapeutically effective amount of a recombinant AAV vector of the invention comprising the pAAV.MHCK7.micro-dystrophin construct nucleotide sequence of SEQ ID NO: 3.

"Fibrosis" refers to the excessive or unregulated deposition of extracellular matrix (ECM) components and abnormal repair processes in tissues upon injury, including skeletal muscle, cardiac muscle, liver, lung, kidney, and pancreas. The ECM components that are deposited include fibronectin and collagen, e.g. collagen 1, collagen 2 or collagen 3.

The invention also provides for a recombinant AAV vector of the invention for use in methods of reducing or preventing fibrosis in a subject suffering from muscular dystrophy comprising administering a therapeutically effective amount of any recombinant AAV vector of the invention.

In another embodiment, the invention provides for a recombinant AAV vector of the invention for use in methods of preventing fibrosis in a subject in need thereof, comprising administering a therapeutically effective amount of a recombinant AAV vector of the invention. For example, any of the rAAV vectors of the invention can be administered to subjects suffering from muscular dystrophy to prevent fibrosis, e.g. the rAAV of the invention expressing a human micro-dystrophin protein administered before fibrosis is observed in the subject. In addition, the rAAV of the invention expressing a human micro-dystrophin gene can be administered to a subject at risk of developing fibrosis, such as those suffering or diagnosed with muscular dystrophy, e.g. DMD. The rAAV of the invention can be administered to the subject suffering from muscular dystrophy in order to prevent new fibrosis in these subjects.

The invention contemplates administering any of the AAV vectors of the invention before fibrosis is observed in the subject. In addition, the rAAV of the invention can be administered to a subject at risk of developing fibrosis, such as those suffering or diagnosed with muscular dystrophy, e.g. DMD. The rAAV of the invention can be administered to the subject suffering from muscular dystrophy who already has developed fibrosis in order to prevent new fibrosis in these subjects.

Also described herein are methods of increasing muscular force and/or muscle mass in a subject suffering from muscular dystrophy comprising administering a therapeutically effective amount of a rAAV vector of the invention expressing a human micro-dystrophin gene. These methods can further comprise the step of administering a rAAV expressing micro-dystrophin.

The invention contemplates the use of any of the AAV vectors of the invention for administration to patients diagnosed with DMD before fibrosis is observed in the subject or before the muscle force has been reduced or before the muscle mass has been reduced.

The invention also contemplates the use of a AAV of the invention for administration to a subject suffering from muscular dystrophy who already has developed fibrosis, in order to prevent new fibrosis in these subjects or to reduce fibrosis in these patients. The invention also provides for the use of any of the rAAV of the invention for administration to the patient suffering from muscular dystrophy who already has reduced muscle force or has reduced muscle mass in order to protect the muscle from further injury.

In any of the methods the subject may be suffering from muscular dystrophy such as DMD or any other dystrophin-associated muscular dystrophy.

In any of the methods the rAAV vector or composition can be administered by intramuscular injection or intravenous injection.

In addition, in any of the methods the rAAV vector or composition can be administered systemically. For example, the rAAV vector or composition can be parenterally administration by injection, infusion, or implantation.

In another embodiment, the invention provides a composition comprising any of the rAAV vectors of the invention for use in reducing fibrosis in a subject in need thereof.

In addition, the invention provides a composition comprising any of the recombinant AAV vectors of the invention for use in preventing fibrosis in a patient suffering from muscular dystrophy.

The invention provides for compositions comprising any of the recombinant AAV vectors of the invention for use in treating muscular dystrophy.

The invention provides for compositions comprising a recombinant AAV vector of the invention comprising the human micro-dystrophin nucleotide sequence of SEQ ID NO: 1 and the MHCK7 promoter sequence of SEQ ID NO: 2 for use in treatment of muscular dystrophy.

The invention provides for a composition comprising a recombinant AAV vector of the invention comprising the pAAV.MHCK7.micro-dystrophin construct comprising the nucleotide sequence of SEQ ID NO: 3 for use in treatment of muscular dystrophy.

Also described herein are compositions comprising any of the rAAV vectors of the invention for increasing muscular force and/or muscle mass in a subject suffering from muscular dystrophy. In a further embodiment, the invention provides for compositions comprising any of the rAAV vectors of the invention for use in treatment of muscular dystrophy.

The compositions of the invention can be formulated for intramuscular injection or intravenous injection. The composition of the invention is also formulated for systemic administration, such as parenterally administration by injection, infusion or implantation.

In addition, any of the compositions can be formulated for administration to a subject suffering from muscular dystrophy such as DMD or any other dystrophin associated muscular dystrophy.

In any of the uses of the invention, the medicament can be formulated for intramuscular injection or intravenous injection. In addition, in any of the uses of the invention, the medicament can be formulated for systemic administration such as parenteral administration by injection, infusion, or implantation.

Any of the medicaments can be prepared for administration to a subject suffering from muscular dystrophy such as DMD or any other dystrophin associated muscular dystrophy.

### BRIEF DESCRIPTION OF THE DRAWINGS

**Figure** 1 illustrates the pAAV.MHCK7.micro-dystrophin construct. In this construct, the cDNA expression cassette is flanked by AAV2 inverted terminal repeat sequences (ITR). The construct is characterized by an in-frame rod deletion (R4-R23), while hinges 1, 2 and 4 (Hi, H₂ and H₄) and the cysteine rich domain remain producing a 138 kDa protein. The expression of the micro-dystrophin protein (3579 bp) is guided by a MHCK7 promoter (795 bp). The intron and 5' UTR are derived from plasmid pCMVβ (Clontech). The micro-dystrophin cassette had a consensus Kozak immediately in front of the ATG start and a small 53 bp synthetic polyA signal for mRNA termination. The human micro-dystrophin cassette contained the (R4-R23/Δ71-78) domains as previously described by Harper et al. (Nature Medicine 8, 253-261 (2002)).

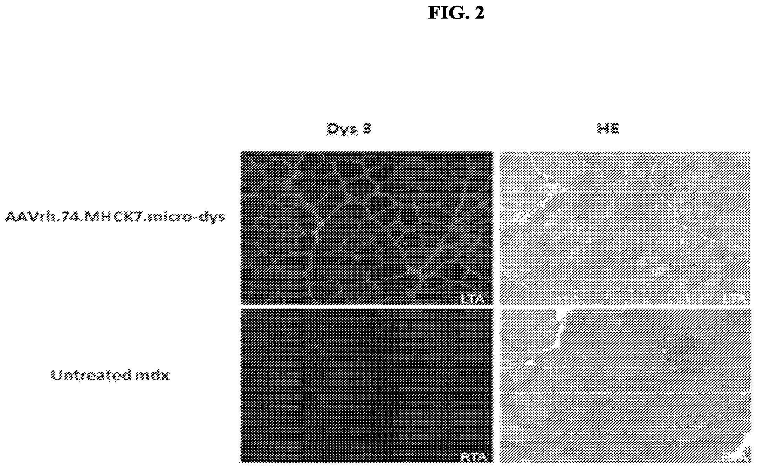

**Figure 2** demonstrates dystrophin protein expression following intramuscular delivery of AAVrh74.MHCK7 construct. The tibialis anterior muscle of mdx mice was injected with 1 × 10¹¹ vg (n=5 per group). Six weeks later the muscles were harvested and stained for dystrophin expression with an N-terminal antibody for dystrophin and hematoxylin and eosin staining.

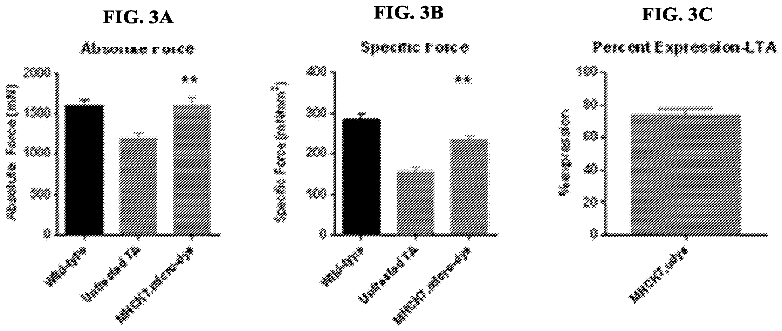

**Figures 3A-3C** provide skeletal muscle force measurements and quantification of micro-dystrophin expression following intramuscular injection of AAVrh74.MHCK7 construct. (A) The tibialis anterior muscle of mdx mice was injected with1 × 10¹¹ vg (n=5) with AAVrh74.MHCK7 construct. Six weeks later the tibialis anterior muscles were harvested and subjected to in vivo force measurements. The dosed cohort had significantly greater force production than untreated mdx controls.

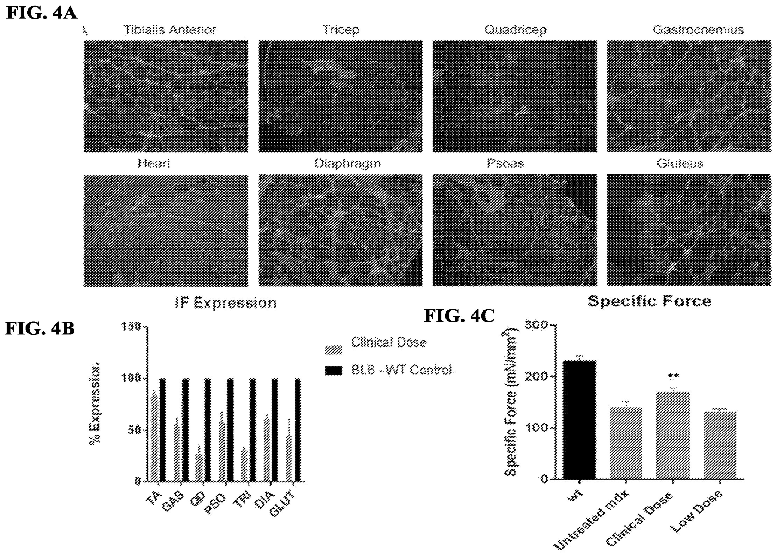

**Figures 4A****-4C** demonstrate widespread transduction of skeletal, diaphragm and cardiac muscle fibers after systemic administration of the AAVrh.74.MHCK7.micro-dys construct. (A) Mdx mice were treated systemically at 6 weeks of age via the tail vein with 6×10¹² vg (2 × 10 ¹⁴ vg/kg) of AAVrh.74.MHCK7.micro-dystrophin following 12 weeks of treatment. (B) Staining for micro-dystrophin demonstrates quantification of the percentage of muscle fibers expressing micro-dystrophin in each tissue. (C) Shows the specific force measured in the diaphragm at the low and high (planned clinical) dose. No significant difference was seen at low dose; however there was significant improvement at the high dose.

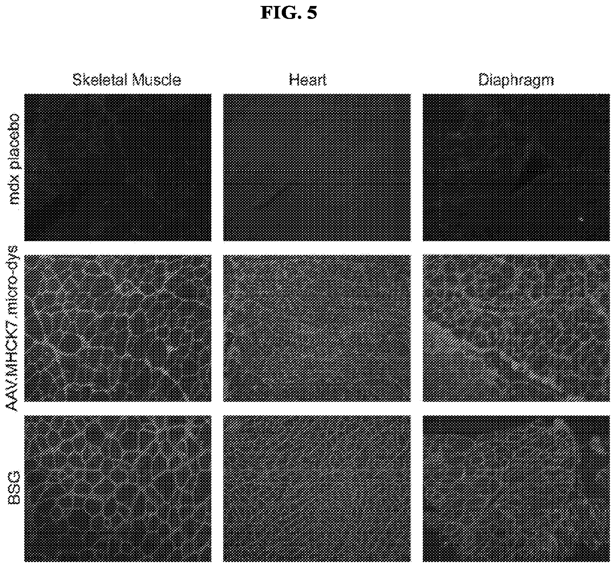

**Figure 5** demonstrates dystrophin protein expression following systemic delivery of AAVrh.74.MHCK7.micro-dystrophin construct. Mdx mice (n=5) were treated systemically starting at 6 weeks of age via the tail vein with 6 × 10 ¹²vg of AAVrh.74.MHCK7.micro-dystrophin. Following 12 weeks of treatment, all muscles were harvested and stained for dystrophin and restoration of DAPC components (beta-sarcoglycan shown).

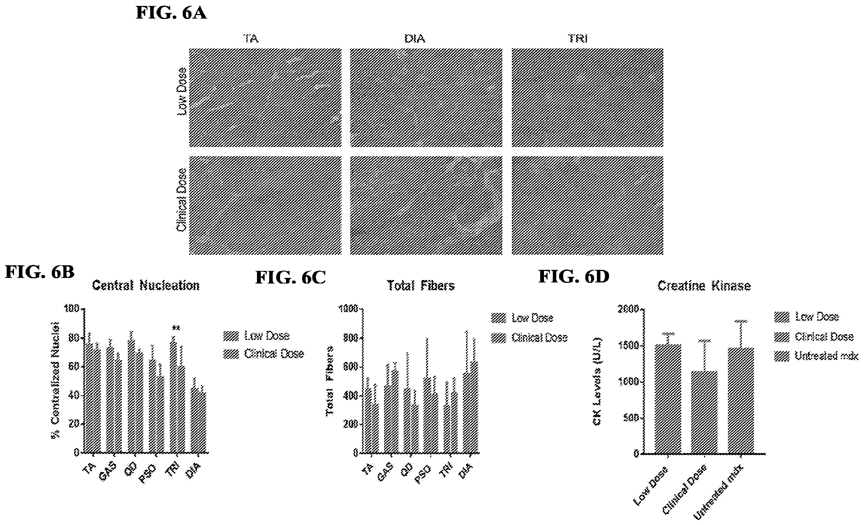

**Figures 6A-6D** demonstrate the toxicology/safety of AAVrh.74.MHCK7. Hematoxylin and eosin (H&E) staining was performed on the following muscle tissues to analyze toxicity: Tibialis anterior (TA), Gastrocnemius (GAS), Quadriceps (QD), Psoas (PSO), Triceps (TRI), and Diaphragm (DIA) (Figure 6A). No toxicity was noted. As an indicator of efficacy, the number of muscle fibers with centrally placed nuclei (CN) was quantified (Figure 6B). CN are indicative of cycles of muscle degeneration and regeneration and thus reduction in CN demonstrates treatment effect. (Figure 6C) demonstrates the total number of fibers is unchanged with treatment. The amount of creatine kinase is provided in (D) showing improvement at high dose. Independent t-tests were used to locate differences (p<.05); Data are reported as means ± SEM.

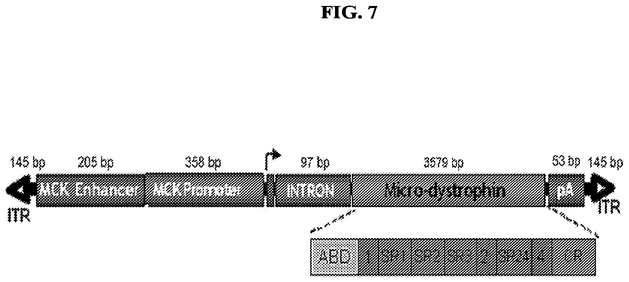

**Figure 7** illustrates the pAAV.MCK.micro-dystrophin plasmid construct.

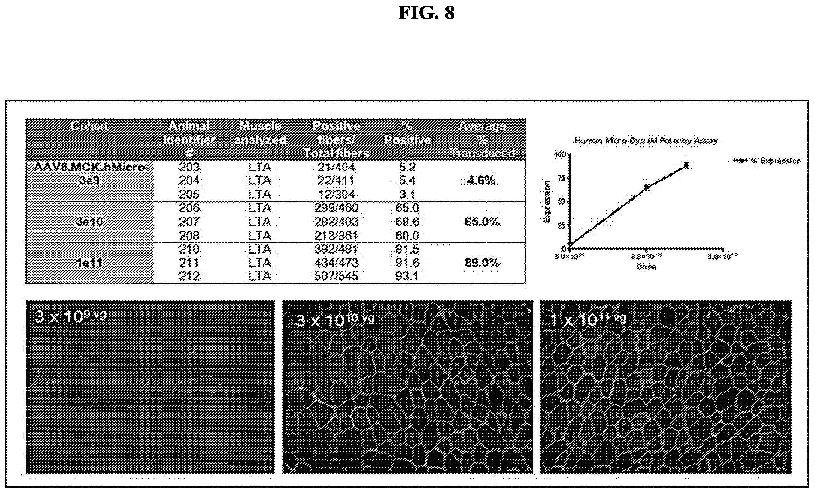

**Figure 8** provides the results of a rAAVrh74.MCK. micro-dystrophin (human) potency assay. The tibialis anterior muscle of mdx mice was injected with 3 × 10⁹, 3 × 10¹⁰, or 1 × 10¹¹ vg (n=3 per group). Four weeks later the muscles were harvested and stained for dystrophin expression with the N-terminal Dys3 antibody. There was a linear correlation between expression and dose with very little expression (no effect level) at 3 × 10⁹ vg and 89% expression at 1 × 10¹¹ vg.

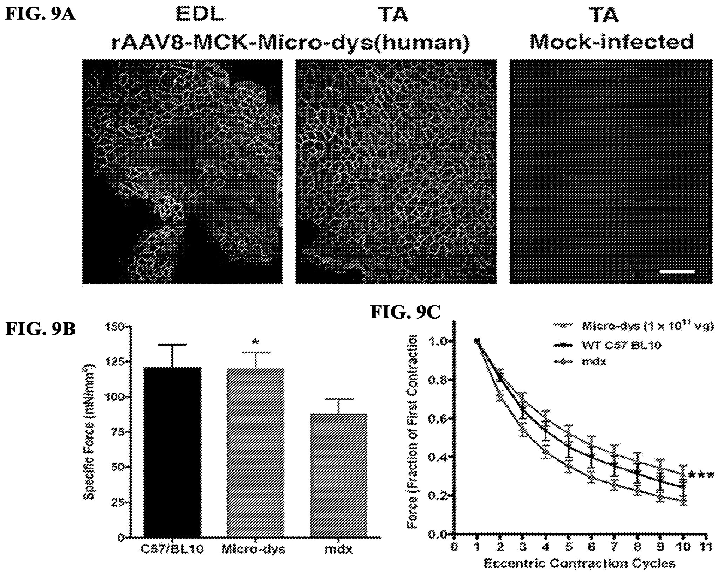

**Figures 9A****-9C** demonstrate that Human micro-dystrophin improves force generation and protection from eccentric contraction induced injury. (A) Dystrophin protein immunostaining in the extensor digitorum longus (EDL) and TA shows expression in a mdx myofibers following rAAVrh.74-MCK-micro-dystrophin (human) injection via the femoral artery. Mock-infected muscle was stained in an identical manner and exposures are time matched. (B) rAAVrh.74-MCK-micro-dystrophin significantly increased normalized specific force relative to mock-treated mdx muscles (P<0.05 vs. mdx). (C) mdx muscles infected with rAAVrh.74-MCK-Micro-dys(human) were compared with mock-infected contralateral mdx EDL muscles and WT (WT C57Bl/10) EDL muscles for force drop during repetitive eccentric contractions at 12 weeks post gene transfer. rAAVrh.74-MCK-microdystrophin (Micro-dys) treatment significantly protected against loss of force compared with mock-treated mdx muscles (P< 0.001 vs. mdx). Errors are SEMs.
**Figure 10** provides the nucleic acid sequence (SEQ ID NO: 3 rAAVrh74.MHCK7. micro-dystrophin).

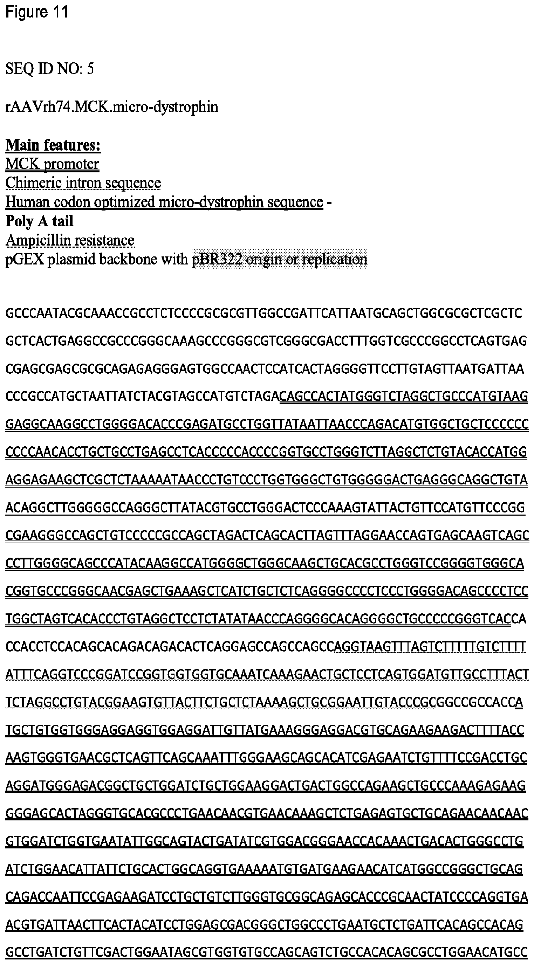

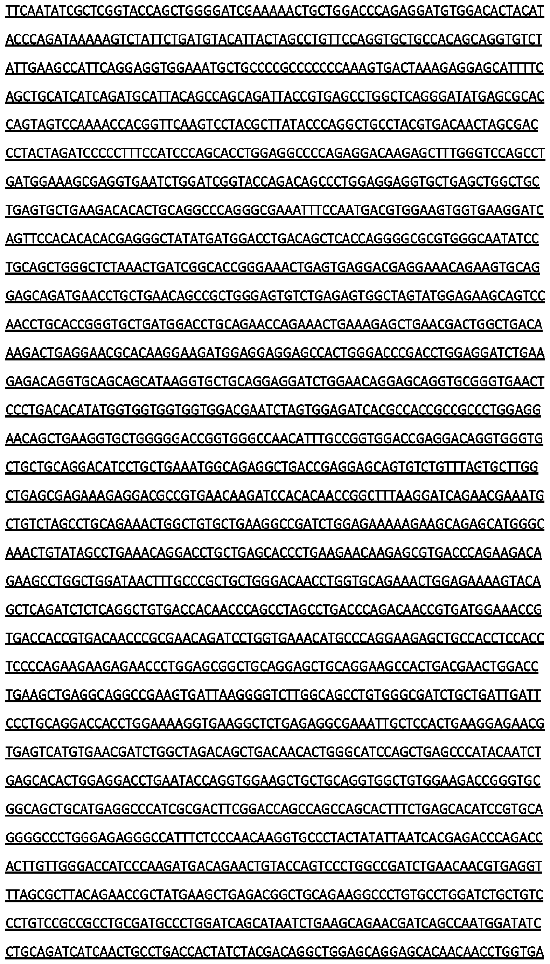

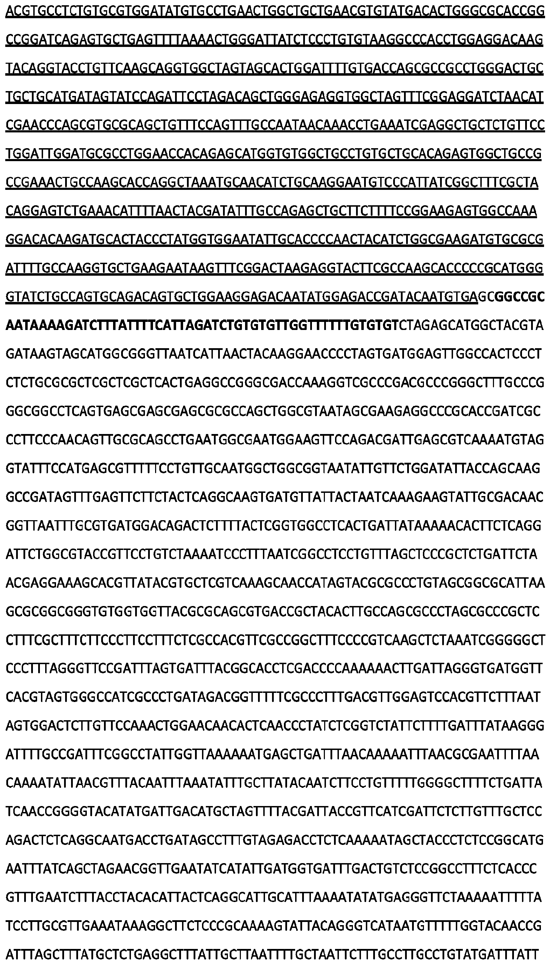

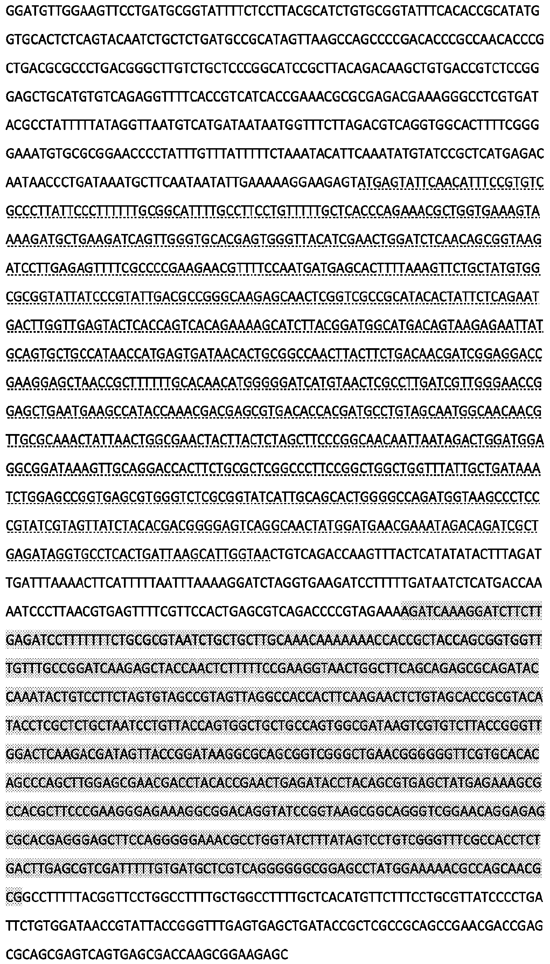

**Figure 11** provides the nucleic acid sequence (SEQ ID NO: 5) rAA Vrh74.MCK.micro-dystrophin.

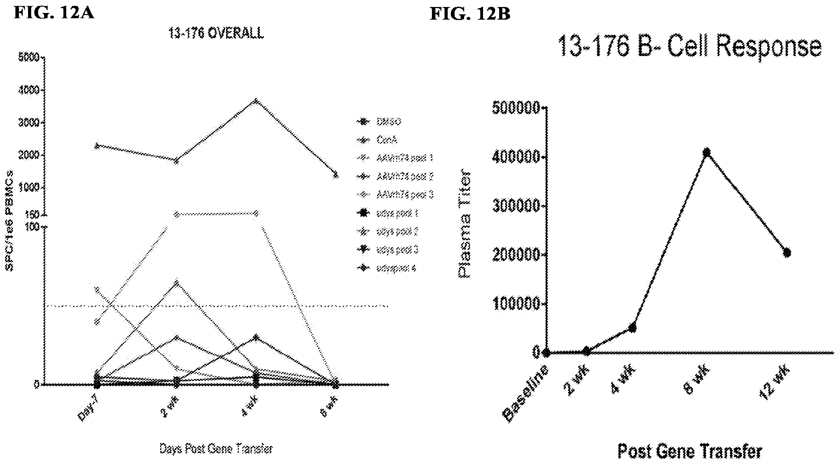

**Figures 12A - 12B** provides the immunological response to systemic delivery of AAVrh74.MHCK7.micro-dystrophin in the non-human primate. (A) ELISpot response to AAV capsid and micro-dystrophin peptide pools. ConA is the positive control and DMSO is the negative control. There were three pools to AAVrh74 and four peptide pools specific to micro-dystrophin. (B) ELISA positive titers of circulating neutralizing antibodies to vector capsid. Serum was isolated from primates biweekly and analyzed for antibody titer. Titer reported corresponds to last dilution at which ratio of response ≥2.

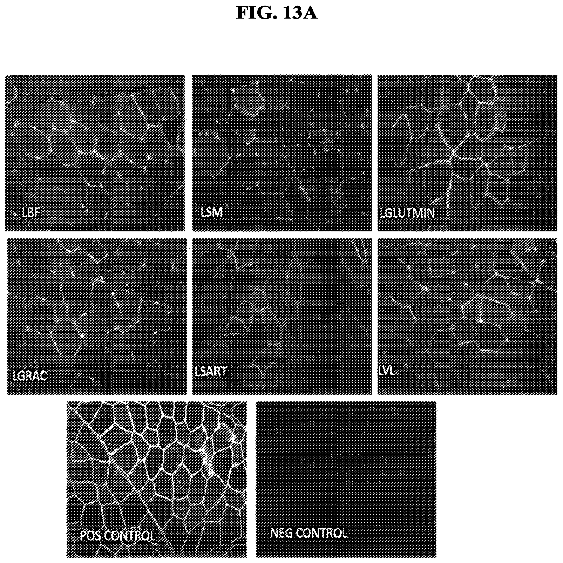

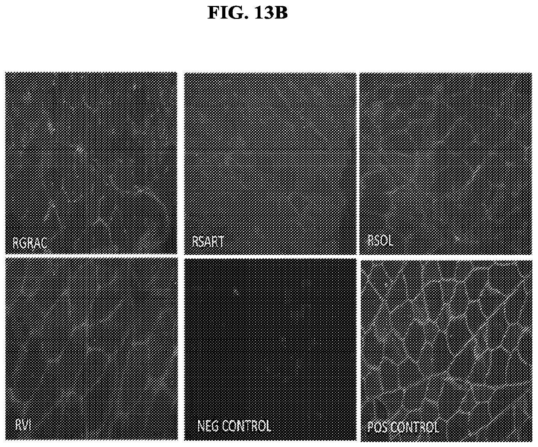

**Figure 13A -B** demonstrates systemic delivery in rhesus macaque with AAVrh74.MHCK7.microdystrophin. Anti-FLAG immunofluorescence staining in the left side muscles demonstrated robust micro-dystrophin expression.

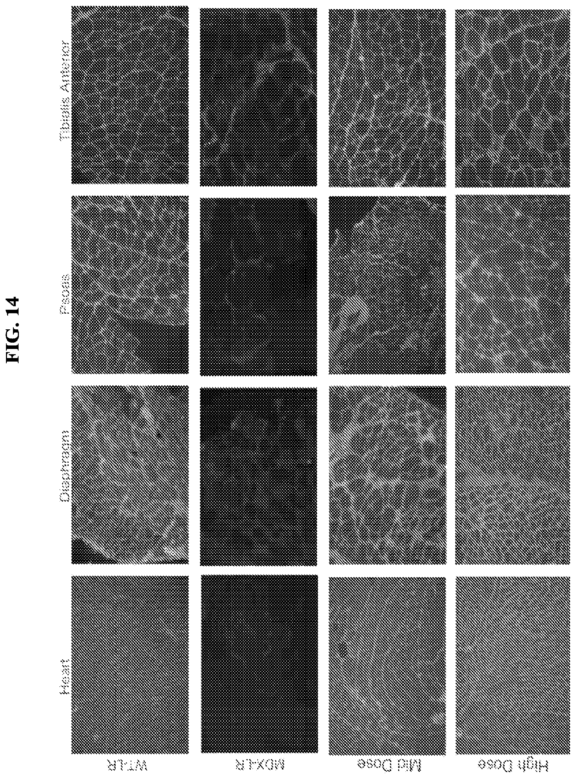

**Figure 14** demonstrates the effect of systemic treatment with rAAVrh74.MHCK7.micro-dystrophin on transgene expression. Immunofluorescence staining for micro-dystrophin using an N-terminal dystrophin antibody in the heart, diaphragm, psoas, and tibialis anterior (TA) demonstrates robust expression in the mid (6e12 vg; 2e14 vg/kg) and high dose (1.2e13 vg; 6e14 vg/kg) treated animals 3 months post-injection. 20x images are shown.

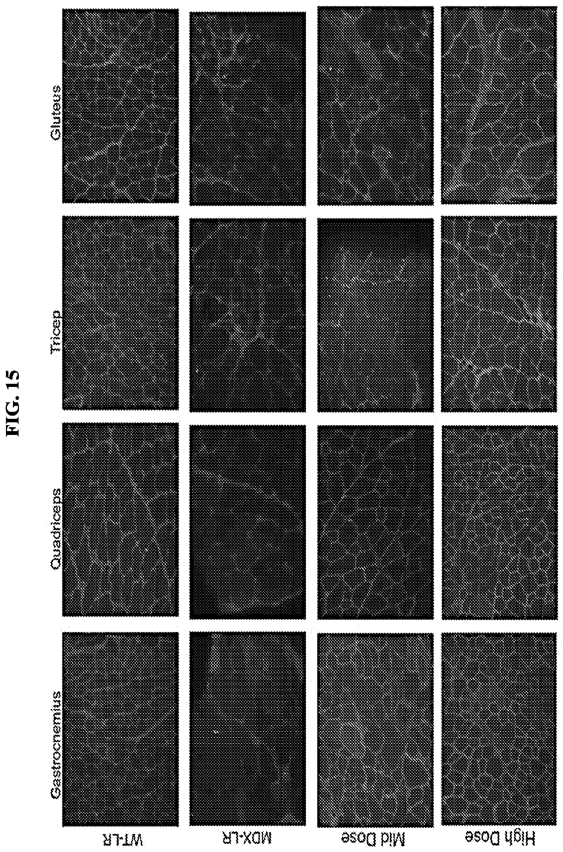

**Figure 15** demonstrates the effect of systemic treatment with rAAVrh74.MHCK7.micro-dystrophin on transgene expression. Immunofluorescence staining for micro-dystrophin using an N-terminal dystrophin antibody in the gastrocnemius, quadriceps, tricep and gluteus demonstrates robust expression in the mid (6e12 vg; 2e14 vg/kg) and highest dose (1.2e13 vg; 6e14 vg/kg) treated animals 3 months post-injection. 20x images are shown.

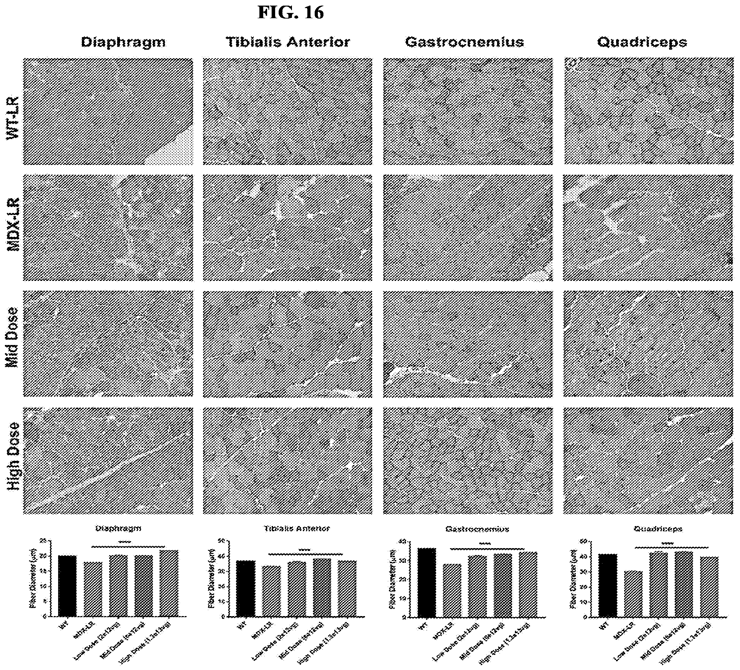

**Figure 16** demonstrates the effect of systemic treatment with rAAVrh74.MHCK7.micro-dystrophin on muscle pathology. (A) H&E stain of diaphragm, tibialis anterior, gastrocnemius, and quadricep muscle from C57BL/6 WT, mdx, and rAAVrh74.MHCK7.micro-dystrophin treated mice (Mid dose-2e14vg/kg; high dose-6e14vg/kg), (B) Quantification of average fiber size demonstrated a normalization of fiber size across all tissue. ****p<0.001, one-way ANOVA; Data are reported as means ± SEM. 20x images are shown.

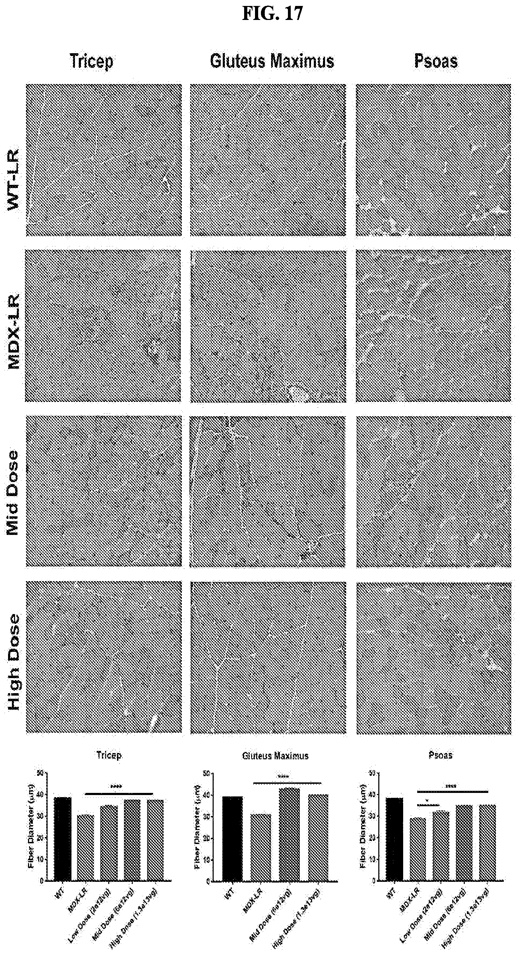

**Figure 17** demonstrates the effect of systemic treatment with rAAVrh74.MHCK7.micro-dystrophin on muscle pathology. (A) H&E stain of tricep, gluteal and psoas muscle from C57BL/6 WT, mdx, and rAAVrh74.MHCK7.micro-dystrophin treated mice (mid dose-2e14vg/kg; high dose-6e14vg/kg), (B) Quantification of average fiber size demonstrated larger fibers in a dose dependent manner. ****p<0.001, one-way ANOVA; Data are reported as means ± SEM. 20x images are shown.

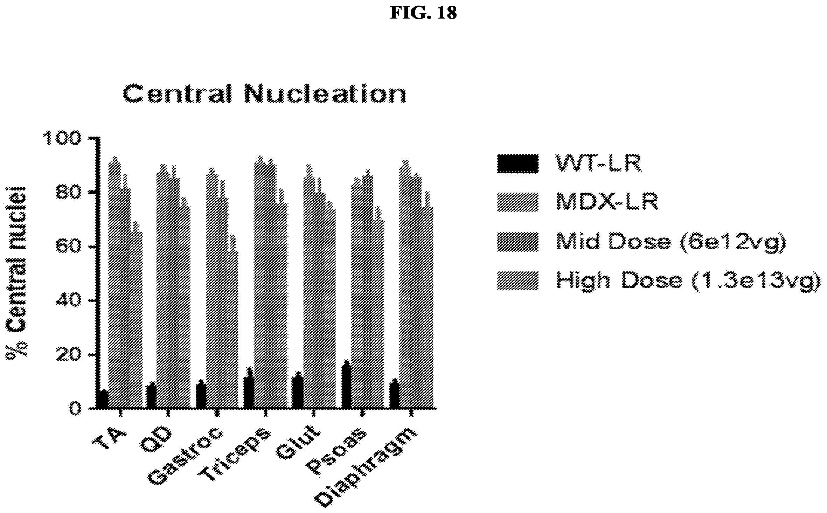

**Figure 18** demonstrates the effect of systemic treatment with rAAVrh74.MHCK7.micro-dystrophin on central nucleation. Dose escalation illustrates reductions in central nucleation in all skeletal muscles and diaphragm. Two-way ANOVA were used to locate differences (p<0.05). Data are reported as means ± SEM.

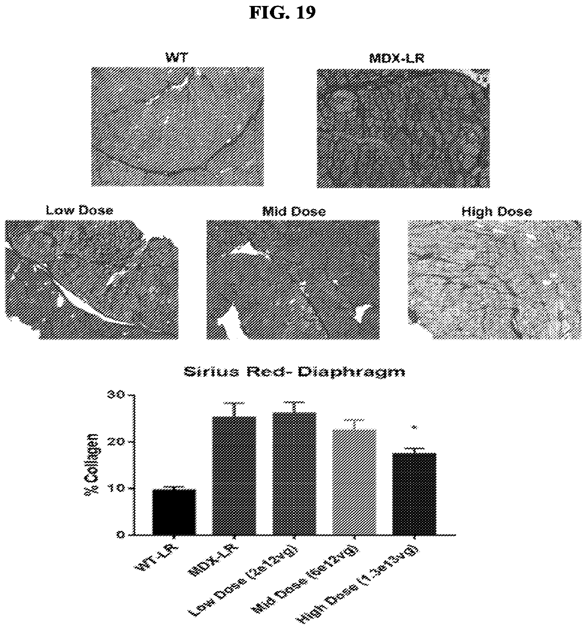

**Figure 19** demonstrates the effect of systemic treatment with rAAVrh74.MHCK7.micro-dystrophin on collagen deposition. Dose escalation illustrates reductions in collagen accumulation (%) in the diaphragm. *p<0.05, one-way ANOVA; Data are reported as means ± SEM. 20x images are shown.

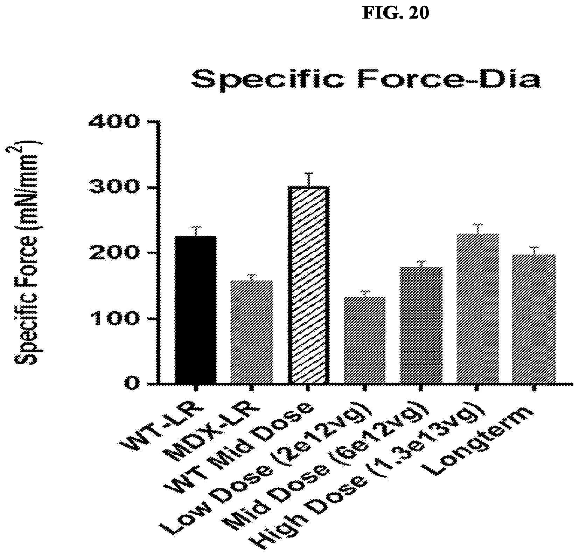

**Figure 20** demonstrates correction of force deficits in the diaphragm. Following 3 or 6 months of treatment, diaphragm muscle strips were harvested to measure specific force (normalized to cross sectional area). Treatment restored force to WT levels. ^{∗}p<0.05. One-way ANOVA was utilized to determine differences from mdx-LR mice.

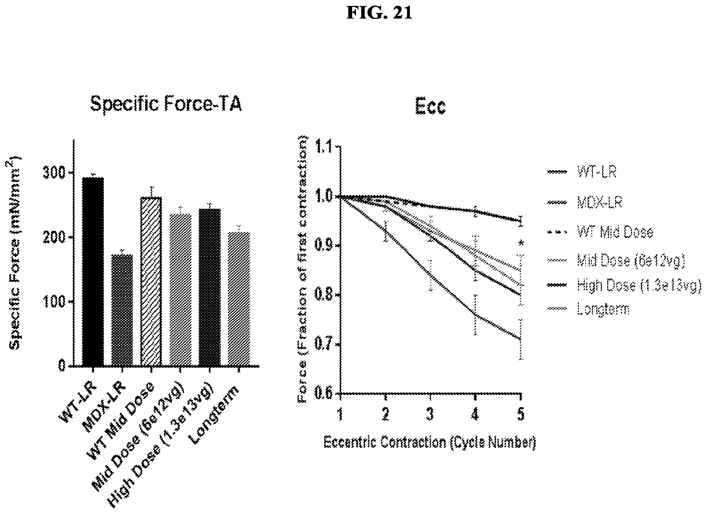

**Figure 21** demonstrates correction of force deficits in the TA. (A) Following 3-6 months of treatment TA muscles were harvested (both Left and Right) to measure specific force (normalized to TA weight). Treatment restored force to WT levels. (B) Treatment rescued TA muscles from fatigue after rigorous protocol of eccentric contractions. ^{∗}p<0.05. One-way ANOVA was utilized to determine differences from mdx-LR mice.

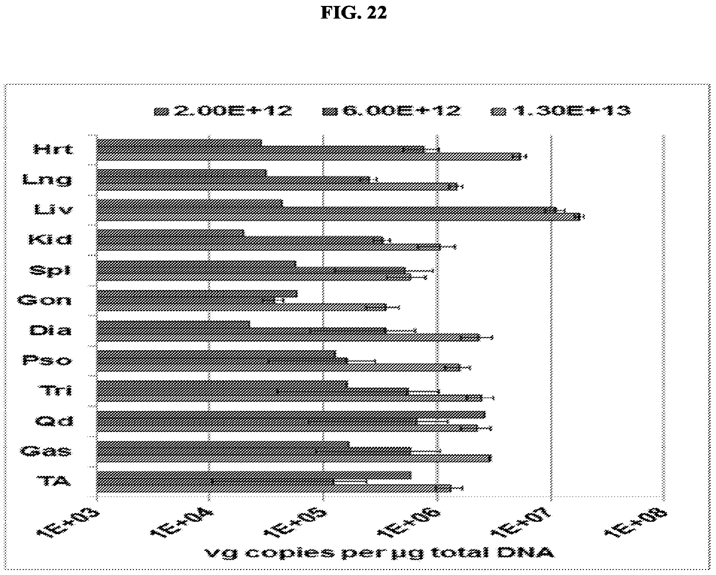

**Figure 22** provides distribution of average vg copies in various tissues from three mdx mice after IV delivery of rAAVrh74.MHCK7.micro-dystrophin.

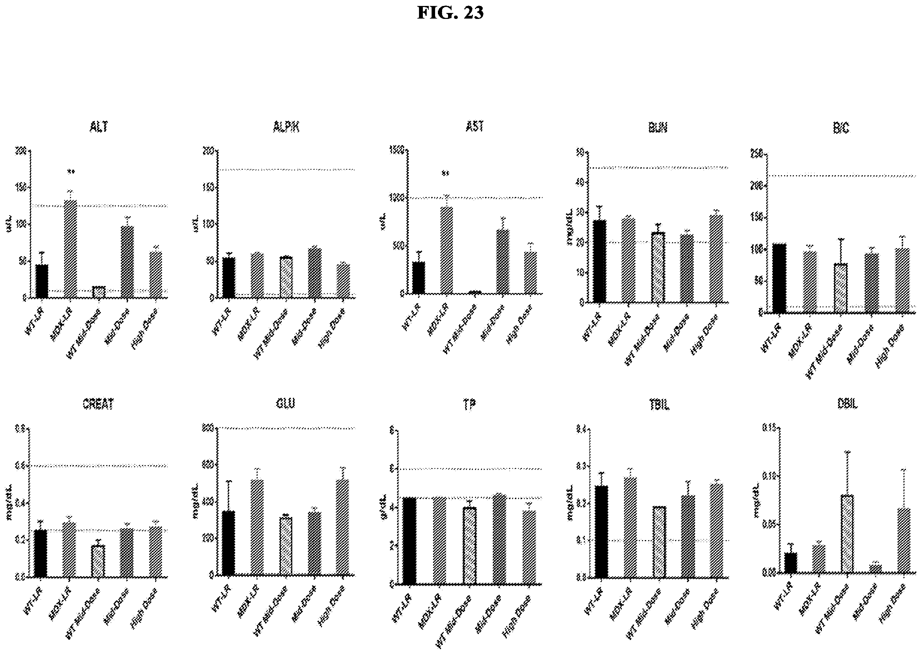

**Figure 23****.** Serum chemistries for ssAAVrh74.MHCK7.micro-dystrophin systemically injected mice and age matched control groups serum chemistries were analyzed by an independent CRO (Charles River Laboratories) which indicate normal values across all chemistries analyzed. The only abnormal values were elevated AST and ALT noted in MDX vehicle treated animals [MDX-LR (lactated ringers)] that was normalized with treatment. AST and ALT are known to be elevated in DMD. ALT = alanine aminotransferase, ALP/K = alkaline phosphatase, AST = aspartate aminotransferase, BUN = blood urea nitrogen, B/C = Blood to creatinine ratio, CREAT = creatine, GLU = glucose, TP= total protein, TBIL = total bilirubin, DBIL = direct bilirubin

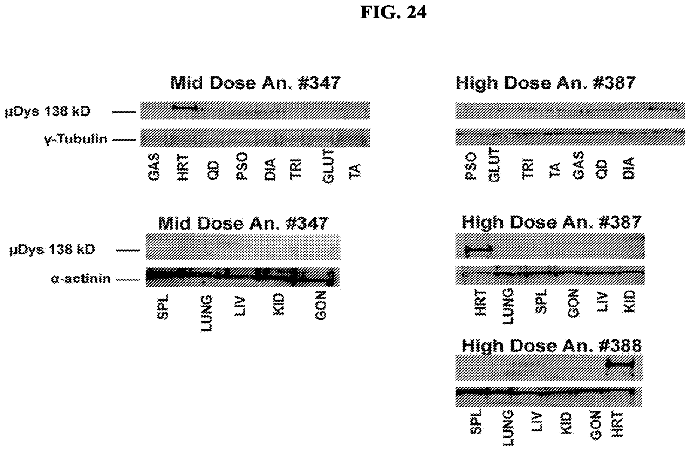

**Figure 24** provides biodistribution western blots on muscles and organs from rAAVrh74.MHCK7.micro-dystrophin systemically injected mdx mice.

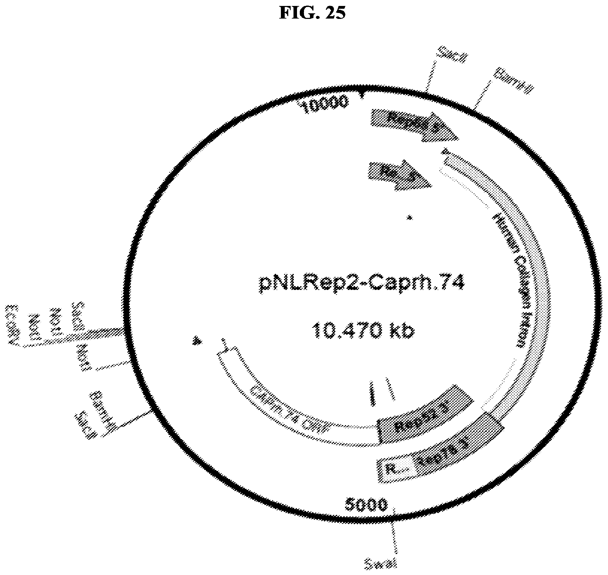

**Figure 25** provides the pNLREP2-Caprh74 AAV helper plasmid map.

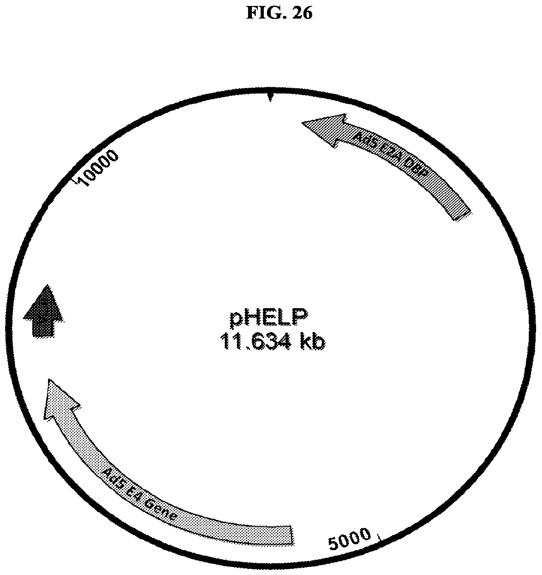

**Figure 26** provides the Ad Helper plasmid pHELP.

### DETAILED DESCRIPTION

The present invention provides for rAAV vectors overexpressing human micro-dystrophin and the rAAV vectors for use in methods of reducing and preventing fibrosis in muscular dystrophy patients. Muscle biopsies taken at the earliest age of diagnosis of DMD reveal prominent connective tissue proliferation. Muscle fibrosis is deleterious in multiple ways. It reduces normal transit of endomysial nutrients through connective tissue barriers, reduces the blood flow and deprives muscle of vascular-derived nutritional constituents, and functionally contributes to early loss of ambulation through limb contractures. Over time, treatment challenges multiply as a result of marked fibrosis in muscle. This can be observed in muscle biopsies comparing connective tissue proliferation at successive time points. The process continues to exacerbate leading to loss of ambulation and accelerating out of control, especially in wheelchair-dependent patients.

Without early treatment including a parallel approach to reduce fibrosis it is unlikely that the benefits of exon skipping, stop-codon read-through, or gene replacement therapies can ever be fully achieved. Even small molecules or protein replacement strategies are likely to fail without an approach to reduce muscle fibrosis. Previous work in aged *mdx* mice with existing fibrosis treated with AAV.micro-dystrophin demonstrated that we could not achieve full functional restoration (Liu, M., et al., Mol Ther 11, 245-256 (2005)). It is also known that progression of DMD cardiomyopathy is accompanied by scarring and fibrosis in the ventricular wall.

As used herein, the term "AAV" is a standard abbreviation for adeno-associated virus. Adeno-associated virus is a single-stranded DNA parvovirus that grows only in cells in which certain functions are provided by a co-infecting helper virus. There are currently thirteen serotypes of AAV that have been characterized. General information and reviews of AAV can be found in, for example, Carter, 1989, Handbook of Parvoviruses, Vol. 1, pp. 169-228, and Berns, 1990, Virology, pp. 1743-1764, Raven Press, (New York). However, it is fully expected that these same principles will be applicable to additional AAV serotypes since it is well known that the various serotypes are quite closely related, both structurally and functionally, even at the genetic level. (See, for example, Blacklowe, 1988, pp. 165-174 of Parvoviruses and Human Disease, J. R. Pattison, ed.; and Rose, Comprehensive Virology 3:1-61 (1974)). For example, all AAV serotypes apparently exhibit very similar replication properties mediated by homologous rep genes; and all bear three related capsid proteins such as those expressed in AAV2. The degree of relatedness is further suggested by heteroduplex analysis which reveals extensive cross-hybridization between serotypes along the length of the genome; and the presence of analogous self-annealing segments at the termini that correspond to "inverted terminal repeat sequences" (ITRs). The similar infectivity patterns also suggest that the replication functions in each serotype are under similar regulatory control.

An "AAV vector" as used herein refers to a vector comprising one or more polynucleotides of interest (or transgenes) that are flanked by AAV terminal repeat sequences (ITRs). Such AAV vectors can be replicated and packaged into infectious viral particles when present in a host cell that has been transfected with a vector encoding and expressing rep and cap gene products.

An "AAV virion" or "AAV viral particle" or "AAV vector particle" refers to a viral particle composed of at least one AAV capsid protein and an encapsidated polynucleotide AAV vector. If the particle comprises a heterologous polynucleotide (i.e. a polynucleotide other than a wild-type AAV genome such as a transgene to be delivered to a mammalian cell), it is typically referred to as an "AAV vector particle" or simply an "AAV vector". Thus, production of AAV vector particle necessarily includes production of AAV vector, as such a vector is contained within an AAV vector particle. **AAV**

The recombinant AAVrh.74 vectors of the invention comprise an MHCK7 muscle-specific promoter/enhancer operably linked to the nucleotide sequence of SEQ ID NO: 1, wherein the recombinant AAVrh.74 vector comprises a 5' AAV2 inverted terminal repeat (ITR), the MHCK7 muscle-specific promoter/enhancer, an SV40 intron, the nucleotide sequence of SEQ ID NO: 1, a synthetic polyadenylation (PolyA) signal and a 3' AAV2 ITR. AAV DNA in the rAAV genomes may be from any AAV serotype for which a recombinant virus can be derived including, but not limited to, AAV serotypes AAVrh.74, AAV-1, AAV-2, AAV-3, AAV-4, AAV-5, AAV-6, AAV-7, AAV-8, AAV-9, AAV-10, AAV-11, AAV-12 and AAV-13. Production of pseudotyped rAAV is disclosed in, for example, WO 01/83692. Other types of rAAV variants, for example rAAV with capsid mutations, are also contemplated. See, for example, Marsic et al., Molecular Therapy, 22(11): 1900-1909 (2014). As noted in the Background section above, the nucleotide sequences of the genomes of various AAV serotypes are known in the art. To promote skeletal muscle specific expression, AAV1, AAV6, AAV8 or AAVrh.74 can be used.

DNA plasmids of the invention comprise rAAV genomes of the invention. The DNA plasmids are transferred to cells permissible for infection with a helper virus of AAV (e.g., adenovirus, E1-deleted adenovirus or herpesvirus) for assembly of the rAAV genome into infectious viral particles. Techniques to produce rAAV particles, in which an AAV genome to be packaged, rep and cap genes, and helper virus functions are provided to a cell are standard in the art. Production of rAAV requires that the following components are present within a single cell (denoted herein as a packaging cell): a rAAV genome, AAV rep and cap genes separate from (i.e., not in) the rAAV genome, and helper virus functions. The AAV rep and cap genes may be from any AAV serotype for which recombinant virus can be derived and may be from a different AAV serotype than the rAAV genome ITRs, including, but not limited to, AAV serotypes AAV-1, AAV-2, AAV-3, AAV-4, AAV-5, AAV-6, AAV-7, AAVrh.74, AAV-8, AAV-9, AAV-10, AAV-11, AAV-12 and AAV-13. Production of pseudotyped rAAV is disclosed in, for example, WO 01/83692.

A method of generating a packaging cell is to create a cell line that stably expresses all the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4666). The packaging cell line is then infected with a helper virus such as adenovirus. The advantages of this method are that the cells are selectable and are suitable for large-scale production of rAAV. Other examples of suitable methods employ adenovirus or baculovirus rather than plasmids to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial. and Immunol., 158:97-129). Various approaches are described in Ratschin et al., Mol. Cell. Biol. 4:2072 (1984); Hermonat et al., Proc. Natl. Acad. Sci. USA, 81:6466 (1984); Tratschin et al., Mol. Cell. Biol. 5:3251 (1985); McLaughlin et al., J. Virol., 62:1963 (1988); and Lebkowski et al., Mol. Cell. Biol., 7:349 (1988). Samulski et al., J. Virol., 63:3822-3828 (1989); U.S. Patent No. 5,173,414; WO 95/13365 and corresponding U.S. Patent No. 5,658.776 ; WO 95/13392; WO 96/17947; PCT/US98/18600; WO 97/09441 (PCT/US96/14423); WO 97/08298 (PCT/US96/13872); WO 97/21825 (PCT/US96/20777); WO 97/06243 (PCT/FR96/01064); WO 99/11764; Perrin et al. Vaccine 13:1244-1250 (1995); Paul et al. Human Gene Therapy 4:609-615 (1993); Clark et al. Gene Therapy 3:1124-1132 (1996); U.S. Patent. No. 5,786,211; U.S. Patent No. 5,871,982; and U.S. Patent. No. 6,258,595.

Described herein are packaging cells that produce infectious rAAV. Packaging cells may be stably transformed cancer cells such as HeLa cells, 293 cells and PerC.6 cells (a cognate 293 line). Packaging cells may be cells that are not transformed cancer cells, such as low passage 293 cells (human fetal kidney cells transformed with E1 of adenovirus), MRC-5 cells (human fetal fibroblasts), WI-38 cells (human fetal fibroblasts), Vero cells (monkey kidney cells) and FRhL-2 cells (rhesus fetal lung cells).

Recombinant AAV (*i.e.*, infectious encapsidated rAAV particles) of the invention comprise a rAAV genome. In exemplary embodiments, the genomes of both rAAV lack AAV rep and cap DNA, that is, there is no AAV rep or cap DNA between the ITRs of the genomes. Examples of rAAV that may be constructed to comprise the nucleic acid molecules of the invention are set out in International Patent Application No. PCT/US2012/047999 (WO 2013/016352).

In an exemplary embodiment, the recombinant AAV vector of the inveiton is produced by the triple transfection method (Xiao et al. ,J Virol 72, 2224-2232 (1998) using the AAV vector plasmids pAAV.MHCK7.micro-dystrophin, pNLRep2-Caprh74 and pHelp, pAAV contains the micro-dystrophin gene expression cassette flanked by AAV2 inverted terminal repeat sequences (ITR). It is this sequence that is encapsidated into AAVrh74 virions. The plasmid contains the micro-dystrophin sequence and the MHCK7 enhancer and core promoter elements of the muscle specific promoter to drive gene expression. The expression cassette also contains an SV40 intron (SD/SA) to promote high-level gene expression and the bovine growth hormone polyadenylation signal is used for efficient transcription termination.

The pNLREP2-Caprh74 is an AAV helper plasmid that encodes the 4 wild-type AAV2 rep proteins and the 3 wild-type AAV VP capsid proteins from serotype rh74. A schematic map of the pNLREP2-Caprh74 plasmid is shown in Figure 25.

The pHELP adenovirus helper plasmid is 11,635 bp and was obtained from Applied Viromics. The plasmid contains the regions of adenovirus genome that are important for AAV replication, namely E2A, E4ORF6, and VA RNA (the adenovirus E1 functions are provided by the 293 cells). The adenovirus sequences present in this plasmid only represents ~40% of the adenovirus genome, and does not contain the *cis* elements critical for replication such as the adenovirus terminal repeats. Therefore, no infectious adenovirus is expected to be generated from such a production system. A schematic map of the pHELP plasmid is shown in Figure 26.

The rAAV may be purified by methods standard in the art such as by column chromatography or cesium chloride gradients. Methods for purifying rAAV vectors from helper virus are known in the art and include methods disclosed in, for example, Clark et al., Hum. Gene Ther., 10(6): 1031-1039 (1999); Schenpp and Clark, Methods Mol. Med., 69 427-443 (2002); U.S. Patent No. 6,566,118 and WO 98/09657.

In another embodiment, the invention contemplates compositions comprising rAAV of the present invention. Compositions of the invention comprise rAAV and a pharmaceutically acceptable carrier. The compositions may also comprise other ingredients such as diluents and adjuvants. Acceptable carriers, diluents and adjuvants are nontoxic to recipients and are preferably inert at the dosages and concentrations employed and include buffers and surfactants such as pluronics.

Titers of rAAV to be administered in methods will vary depending, for example, on the particular rAAV, the mode of administration, the treatment goal, the individual, and the cell type(s) being targeted, and may be determined by methods standard in the art. Titers of rAAV may range from about 1×10⁶, about 1×10⁷, about 1×10⁸, about 1×10⁹, about 1×10¹⁰, about 1×10¹¹, about 1×10¹², about 1×10¹³ to about 1×10¹⁴ or more DNase resistant particles (DRP) per ml. Dosages may also be expressed in units of viral genomes (vg).

Methods of transducing a target cell with rAAV, *in vivo* or *in vitro,* are described herein. The *in vivo* methods comprise the step of administering an effective dose, or effective multiple doses, of a composition comprising a rAAV of the invention to an animal (including a human being) in need thereof. If the dose is administered prior to development of a disorder/disease, the administration is prophylactic. If the dose is administered after the development of a disorder/disease, the administration is therapeutic. In embodiments of the invention, an effective dose is a dose that alleviates (eliminates or reduces) at least one symptom associated with the disorder/disease state being treated, that slows or prevents progression to a disorder/disease state, that slows or prevents progression of a disorder/disease state, that diminishes the extent of disease, that results in remission (partial or total) of disease, and/or that prolongs survival. An example of a disease contemplated for prevention or treatment with methods of the invention is DMD.

Combination therapies are also contemplated by the invention. Combination as used herein includes both simultaneous treatment and sequential treatments. Combinations of methods of the invention with standard medical treatments (e.g., corticosteroids) are specifically contemplated, as are combinations with novel therapies.

Administration of an effective dose of the compositions may be by routes standard in the art including, but not limited to, intramuscular, parenteral, intravenous, oral, buccal, nasal, pulmonary, intracranial, intraosseous, intraocular, rectal, or vaginal. Route(s) of administration and serotype(s) of AAV components of the rAAV (in particular, the AAV ITRs and capsid protein) of the invention may be chosen and/or matched by those skilled in the art taking into account the infection and/or disease state being treated and the target cells/tissue(s) that are to express the micro-dystrophin protein.

The invention provides for local administration and systemic administration of an effective dose of rAAV and compositions of the invention. For example, systemic administration is administration into the circulatory system so that the entire body is affected. Systemic administration includes enteral administration such as absorption through the gastrointestinal tract and parenteral administration through injection, infusion or implantation.

In particular, actual administration of rAAV of the present invention may be accomplished by using any physical method that will transport the rAAV recombinant vector into the target tissue of an animal. Administration according to the invention includes, but is not limited to, injection into muscle and injection into the bloodstream. Simply resuspending a rAAV in phosphate buffered saline has been demonstrated to be sufficient to provide a vehicle useful for muscle tissue expression, and there are no known restrictions on the carriers or other components that can be co-administered with the rAAV (although compositions that degrade DNA should be avoided in the normal manner with rAAV). Capsid proteins of a rAAV may be modified so that the rAAV is targeted to a particular target tissue of interest such as muscle. See, for example, WO 02/053703. Pharmaceutical compositions can be prepared as injectable formulations or as topical formulations to be delivered to the muscles by transdermal transport. Numerous formulations for both intramuscular injection and transdermal transport have been previously developed and can be used in the practice of the invention. The rAAV can be used with any pharmaceutically acceptable carrier for ease of administration and handling.

The dose of rAAV to be administered in methods disclosed herein will vary depending, for example, on the particular rAAV, the mode of administration, the treatment goal, the individual, and the cell type(s) being targeted, and may be determined by methods standard in the art. Titers of each rAAV administered may range from about 1×10⁶, about 1×10⁷, about 1×10⁸, about 1×10⁹, about 1×10¹⁰, about 1×10¹¹, about 1×10¹², about 1×10¹³, about 1×10¹⁴, or to about 1×10¹⁵ or more DNase resistant particles (DRP) per ml. Dosages may also be expressed in units of viral genomes (vg) (*i.e.,* 1×10⁷ vg, 1×10⁸ vg, 1×10⁹ vg, 1×10¹⁰ vg, 1×10¹¹ vg, 1×10¹² vg, 1×10¹³ vg, 1×10¹⁴ vg, 1×10¹⁵ respectively). Dosages may also be expressed in units of viral genomes (vg) per kilogram (kg) of bodyweight (*i.e*., 1×10¹⁰ vg/kg, 1×10¹¹ vg/kg, 1×10¹² vg/kg, 1×10¹³ vg/kg, 1×10¹⁴ vg/kg, 1×10¹⁵ vg/kg respectively). Methods for titering AAV are described in Clark et al., Hum. Gene Ther., 10: 1031-1039 (1999).

In particular, actual administration of rAAV of the present invention may be accomplished by using any physical method that will transport the rAAV recombinant vector into the target tissue of an animal. Administration according to the invention includes, but is not limited to, injection into muscle and injected into the bloodstream. Simply resuspending a rAAV in phosphate buffered saline has been demonstrated to be sufficient to provide a vehicle useful for muscle tissue expression, and there are no known restrictions on the carriers or other components that can be co-administered with the rAAV (although compositions that degrade DNA should be avoided in the normal manner with rAAV). Capsid proteins of a rAAV may be modified so that the rAAV is targeted to a particular target tissue of interest such as muscle. See, for example, WO 02/053703. Pharmaceutical compositions can be prepared as injectable formulations or as topical formulations to be delivered to the muscles by transdermal transport. Numerous formulations for both intramuscular injection and transdermal transport have been previously developed and can be used in the practice of the invention. The rAAV can be used with any pharmaceutically acceptable carrier for ease of administration and handling.

For purposes of intramuscular injection, solutions in an adjuvant such as sesame or peanut oil or in aqueous propylene glycol can be employed, as well as sterile aqueous solutions. Such aqueous solutions can be buffered, if desired, and the liquid diluent first rendered isotonic with saline or glucose. Solutions of rAAV as a free acid (DNA contains acidic phosphate groups) or a pharmacologically acceptable salt can be prepared in water suitably mixed with a surfactant such as hydroxpropylcellulose. A dispersion of rAAV can also be prepared in glycerol, liquid polyethylene glycols and mixtures thereof and in oils. Under ordinary conditions of storage and use, these preparations contain a preservative to prevent the growth of microorganisms. In this connection, the sterile aqueous media employed are all readily obtainable by standard techniques well-known to those skilled in the art.

The pharmaceutical carriers, diluents or excipients suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating actions of microorganisms such as bacteria and fungi. The carrier can be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride. Prolonged absorption of the injectable compositions can be brought about by use of agents delaying absorption, for example, aluminum monostearate and gelatin.

Sterile injectable solutions are prepared by incorporating rAAV in the required amount in the appropriate solvent with various other ingredients enumerated above, as required, followed by filter sterilization. Generally, dispersions are prepared by incorporating the sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique that yield a powder of the active ingredient plus any additional desired ingredient from the previously sterile-filtered solution thereof.

Transduction with rAAV may also be carried out *in vitro.* In one embodiment, desired target muscle cells are removed from the subject, transduced with rAAV and reintroduced into the subject. Alternatively, syngeneic or xenogeneic muscle cells can be used where those cells will not generate an inappropriate immune response in the subject.

Suitable methods for the transduction and reintroduction of transduced cells into a subject are known in the art. In one embodiment, cells can be transduced *in vitro* by combining rAAV with muscle cells, *e.g.,* in appropriate media, and screening for those cells harboring the DNA of interest using conventional techniques such as Southern blots and/or PCR, or by using selectable markers. Transduced cells can then be formulated into pharmaceutical compositions, and the composition introduced into the subject by various techniques, such as by intramuscular, intravenous, subcutaneous and intraperitoneal injection, or by injection into smooth and cardiac muscle, using *e.g.,* a catheter.

Transduction of cells with rAAV of the invention results in sustained expression of the micro-dystrophin protein. Thus methods of administering/delivering rAAV which express micro-dystrophin protein to an animal, preferably a human being are provided. These methods include transducing tissues (including, but not limited to, tissues such as muscle, organs such as liver and brain, and glands such as salivary glands) with one or more rAAV of the present invention. Transduction may be carried out with gene cassettes comprising tissue specific control elements. For example, described herein are methods of transducing muscle cells and muscle tissues directed by muscle specific control elements, including, but not limited to, those derived from the actin and myosin gene families, such as from the myoD gene family (See Weintraub et al., Science, 251: 761-766 (1991)), the myocyte-specific enhancer binding factor MEF-2 (Cserjesi and Olson, Mol Cell Biol 11: 4854-4862 (1991)), control elements derived from the human skeletal actin gene (Muscat et al., Mol Cell Biol, 7: 4089-4099 (1987)), the cardiac actin gene, muscle creatine kinase sequence elements (See Johnson et al., Mol Cell Biol, 9:3393-3399 (1989)) and the murine creatine kinase enhancer (mCK) element, control elements derived from the skeletal fast-twitch troponin C gene, the slow-twitch cardiac troponin C gene and the slow-twitch troponin I gene: hypoxia-inducible nuclear factors (Semenza et al., Proc Natl Acad Sci USA, 88: 5680-5684 (1991)), steroid-inducible elements and promoters including the glucocorticoid response element (GRE) (See Mader and White, Proc. Natl. Acad. Sci. USA 90: 5603-5607 (1993)), and other control elements.

Muscle tissue is an attractive target for *in vivo* DNA delivery, because it is not a vital organ and is easy to access. The invention contemplates sustained expression of microdystrophin from transduced myofibers.

By "muscle cell" or "muscle tissue" is meant a cell or group of cells derived from muscle of any kind (for example, skeletal muscle and smooth muscle, *e.g.* from the digestive tract, urinary bladder, blood vessels or cardiac tissue). Such muscle cells may be differentiated or undifferentiated, such as myoblasts, myocytes, myotubes, cardiomyocytes and cardiomyoblasts.

The term "transduction" is used to refer to the administration/delivery of the coding region of the micro-dystrophin to a recipient cell either *in vivo* or *in vitro,* via a replication-deficient rAAV of the invention resulting in expression of micro-dystrophin by the recipient cell.

Thus, methods of administering an effective dose (or doses, administered essentially simultaneously or doses given at intervals) of rAAV that encode micro-dystrophin to a patient in need thereof are provided.

### EXAMPLES

### Example 1

### Generation of the pAAV.MHCK7.micro-dystrophin construct

The pAAV.MHCK7.micro-dystrophin plasmid contains a human micro-dystrophin cDNA expression cassette flanked by AAV2 inverted terminal repeat sequences (ITR) (see Fig. 1). The micro-dystrophin construct was characterized by an in-frame rod deletion (R4-R23), while hinges 1, 2 and 4 and cysteine rich domain remain producing a 138 kDa protein. The expression of the micro-dystrophin protein (3579 bp) was guided by a MHCK7 promoter (795 bp). The plasmid was constructed from the pAAV.MCK.micro-dystrophin plasmid by removing the MCK promoter and inserting the MHCK7 promoter. After the core promoter, the 53 bp endogenous mouse MCK Exon1 (untranslated) is present for efficient transcription initiation, followed by the SV40 late 16S/19S splice signals (97 bp) and a small 5'UTR (61 bp). The intron and 5' UTR are derived from plasmid pCMVβ (Clontech). The micro-dystrophin cassette had a consensus Kozak immediately in front of the ATG start and a small 53 bp synthetic polyA signal for mRNA termination. The human micro-dystrophin cassette contained the (R4-R23/Δ71-78) domains as previously described by Harper et al. (Nature Medicine 8, 253-261 (2002)). The complementary DNA was codon optimized for human usage and synthesized by GenScript (Piscataway, NJ) (Mol Ther 18, 109-117 (2010)). The only viral sequences included in this vector were the inverted terminal repeats of AAV2, which are required for both viral DNA replication and packaging. The micro-dystrophin cassette has a small 53 bp synthetic polyA signal for mRNA termination.

Previous studies have validated cardiac expression using MHCK7 promoter (Salva et al. Mol Ther 15, 320-329 (2007) and AAVrh74 achieving skeletal, diaphragm, and cardiac muscle expression (Sondergaard et al. Annals of clinical and Transl Neurology 2, 256-270 (2015)), The sequence of construct of Fig. 1 was encapsidated into AAVrh.74 virions. The molecular clone of the AAVrh.74 serotype was cloned from a rhesus macaque lymph node and is described in in Rodino-Klapac et al. Journal of Translational medicine 5, 45 (2007).

**Table 1 shows the molecular features of the plasmid pAAV.MHCK7.micro-dystrophin (SEQ ID NO: 3)**

| **Table 1. Molecular Features of plasmid pAAV.MHCK7.micro-dystrophin** | | | | |
|---|---|---|---|---|
| TYPE | START | END | NAME | DESCRIPTION |
| REGION | 7 | 116 | 5' ITR | Wild-type AAV2 inverted terminal repeat |
| REGION | 236 | 1036 | MHCK7 | Mouse myosin heavy chain complex - E box muscle creatine kinase fusion enhancer/promoter |
| REGION | 1046 | 1195 | Chimeric intron | 5' donor site from human β-globin gene and the branchpoint and 3' splice acceptor site from IgG heavy chain variable region |
| GENE | 1206 | 4786 | huDys cDNA | Human micro-dystrophin cDNA |
| REGION | 4787 | 4842 | PolyA | Synthetic PolyA |
| REGION | 4933 | 5042 | 3' ITR | Wild-type AAV2 inverted terminal repeat |
| GENE | 6808 | 7668 | AmpR | β-lactamase gene |
| REGION | 7823 | 8442 | Ori | Plasmid origin of replication |

### Example 2

### Intramuscular Expression Studies Using rAAV.MHCK7.micro-dystrophin

Expression studies were conducted with the human micro-dystrophin construct (rAAVrh74.MHCK7. micro-dystrophin; described in Example 1) by intramuscular injection. The tibialis anterior muscle of mdx mice (spontaneous Dmd^{mdx} mutant mice that do not express dystrophin) were injected with 1 × 10¹¹ vg of the cassette (n=5 per group). Six weeks later the muscles were harvested and stained for dystrophin (Dys3) expression with an N-terminal antibody for dystrophin and hematoxylin and eosin (HE) staining. Figure 2 shows diffuse gene expression and reduction in centrally located nuclei with 1 × 10¹¹ vg dose compared to the untreated muscle. Furthermore, a decrease in central nucleation with an increase in average fibers/frame was observed following treatment with micro-dystrophin construct. Expression levels of the rAAVrh74.MHCK7. micro-dystrophin construct were quantified at about 73%.

In addition to measuring micro-dystrophin localization and expression levels, skeletal muscle force was measured following intramuscular injection of the cassette. Intramuscular expression of pAAV.MHCK7.micro-dystrophin construct resulted in significantly greater absolute and specific force production compared with untreated controls (Figures 3A and 3B, respectfully).

### Example 3

### Systemic Delivery of rAAVrh.74.MHCK7.micro-dystrophin to mdx mice

Cohorts of mdx mice were injected via tail vein with either 2 ×10¹² vg (8×10¹³ vg/kg) or high dose (planned clinical dose) 6 ×10¹² vg (2×10¹⁴ vg/kg) of rAAVrh.74.MHCK7.micro-dystrophin at 6 weeks of age. Following 12 weeks of treatment, all muscles were harvested and stained for dystrophin and restoration of DAPC components. Systemically injected (tail vein) mice showed high levels of staining of dystrophin throughout all muscles. Figure 4A represents the widespread transduction of skeletal, diaphragm and cardiac muscle fibers after a 6×10¹² vg (2 × 10 ¹⁴ vg/kg) systemic dose. Figure 4B shows quantification of the percentage of muscle fibers expressing micro-dystrophin in each tissue. Finally the diaphragm was tested for functional improvement (Figure 4C). No significant difference was seen at low dose; however there was significant improvement at the high dose. Importantly, Figure 5 demonstrates other components of the DAPC were completely restored following micro-dystrophin delivery. Shown is Beta-sarcoglycan (B-SG).

The toxicology/safety of AAVrh.74.MHCK7.micro-dystrophin are were evaluated by administering the vector via intravenous (i.v.) injection to the tail vein of mdx mice per Table 2. There was no evidence of toxicity in any of the muscle tissues analyzed including: Tibialis anterior (TA), Gastrocnemius (GAS), Quadriceps (QD), Psoas (PSO), Triceps (TRI), and Diaphragm (DIA) (Figure 6A and 6B). The number of centrally placed nuclei were decreased with the high dose 6×10¹² vg (2 × 10 ¹⁴ vg/kg). Historically, central nucleation of skeletal muscles in untreated age matched mdx mice are on average ~80%. Finally, the preliminary data from a small sample size (n=3) demonstrates a decreased level of CK release (U/L) in serum of high dose treated mice (D). Independent t-tests were used to locate differences (p<.05); Data are reported as means ± SEM.

**Table 2. Outline of toxicology/safety study of rAAVrh.74.MHCK7.micro-dystrophin in mice.**

| **Cohort Number** | | **Study Agent** | **Dose (vg/kg)** | **Treatment Day 0** | **Follow-up Day 1** | **Sacrificial EndPoint** | |
|---|---|---|---|---|---|---|---|
| | | | | | | **Week 6** | **Extra** |
| (1) | Low Dose | AAVrh.74.MH CK7.Micro-dys | 8.0 ×10¹³ | Single *i.v.* injection to the tail vein of mdx mice | 24 h Weight, Clinical Observations | 5M | +2 |
| (2) | High Dose | AAVrh.74.MH CK7.Micro-dys | 2.0 ×10¹⁴ | | | 5M | +2 |
| (3) | Control | Vehicle (LRS) | 0 | | | 5M | +2 |
| **TOTAL MICE** | | | | | | | **N = 21** |

### Example 4

### Generation of the pAAV.MCK.micro-dystrophin construct

The pAAV.MCK.micro-dystrophin plasmid was constructed by inserting the MCK expression cassette driving a codon optimized human micro-dystrophin cDNA sequence into the AAV cloning vector psub201 (Samulski et al., J. Virol. 61(10):3096-3101). A muscle-specific regulatory element was included in the construct to drive muscle-specific gene expression. This regulatory element comprised the mouse MCK core enhancer (206 bp) fused to the 351 bp MCK core promoter (proximal). After the core promoter, the construct comprises the 53 bp endogenous mouse MCK Exon1 (untranslated) for efficient transcription initiation, followed by the SV40 late 16S/19S splice signals (97 bp) and a small 5'UTR (61 bp). The intron and 5' UTR was derived from plasmid pCMVβ (Clontech). The micro-dystrophin cassette has a consensus Kozak immediately in front of the ATG start and a small 53 bp synthetic polyA signal for mRNA termination. The human micro-dystrophin cassette contains the (R4-R23/Δ71-78) domains as previously described by Harper et al. Nat. Med. 8(3):253-61, 2002

The pAAV.MCK.micro-dystrophin plasmid contained the human micro-dystrophin cDNA expression cassette flanked by AAV2 inverted terminal repeat sequences (ITR) (see Fig. 7). This sequence was encapsidated into AAVrh.74 virions. The molecular clone of the AAVrh.74 serotype was cloned from a rhesus macaque lymph node and is described in Rodino-Klapac et al. Journal of Tran. Med. 45 (2007).

### Example 5

### Potency and Dose Analysis Using rAAV.MCK.micro-dystrophin

Expression studies were conducted with the human micro-dystrophin construct (rAAV.MCK.micro-dystrophin; described in Example 1) by intramuscular injection. The tibialis anterior (TA) muscle of mdx mice (spontaneous Dmd^{mdx} mutant mice that do not express dystrophin) were injected with 3 × 10⁹, 3 × 10¹⁰, or 1 × 10¹¹ vg (n=3 per group). Four weeks later the muscles were harvested and stained for dystrophin expression using an antibody specific for the N-terminal Dys3 and hematoxylin and eosin (HE) staining. Figure 8 show a linear correlation between expression and dose where very little expression (no effect level) at 3 × 10⁹ vg and 89% expression at 1 × 10¹¹ vg.

### Example 6

### Vascular Delivery of rAAV.MCK.micro-dystrophin to mdx Mice

Using an isolated limb perfusion model (Rodino-Klapac et al., J. Trans. Med. 5(45): 1-11, 2007), mdx mice (n=10) were injected with 1 × 10¹¹ vg of rAAVrh.74.MCK.micro-dystrophin via the femoral artery and performed outcomes analysis was carried out. Three months post gene transfer, lower limb muscles were harvested and efficacy studies demonstrated significant improvement in both force and resistance to eccentric contraction induced injury (Figure 9).

Dystrophin protein immunostaining in the extensor digitorum longus (EDL) muscle and TA muscle shows expression in a mdx myofibers following rAAVrh.74-MCK-micro-dystrophin treatment (Fig. 9A). Mock-infected muscle was stained in an identical manner and exposures were time matched. Fig. 9B demonstrates that rAAVrh.74-MCK-micro-dystrophin significantly increased normalized specific force relative to mock-treated mdx muscles (P<0.05 vs. mdx). In addition, the mdx muscles infected with rAAVrh.74-MCK-micro-dystrophin (human) were compared with mock-infected contralateral mdx EDL muscles (blue) and Wild Type (WT C57Bl/10) EDL muscles for force drop during repetitive eccentric contractions at 12 weeks post gene transfer (Fig. 9C). It was found that rAAVrh.74-MCK-microdystrophin (Micro-dys) treatment significantly protected against loss of force compared with mock-treated mdx muscles (P< 0.001 vs. mdx).

### Example 7

### Primate Studies

In order to apply pre-clinical finds in mice to a clinical paradigm, a non-human primate (NHP) was dosed systemically in order to evaluate safety and efficacy for future clinical trials. The effect of 2×10¹⁴ vg total dose of AAVrh74.MHCK7.micro-dystrophin.FLAG delivered intravenously through the cephalic vein was studied in a non-human primate. This dose was proportional (based on animal weight) to the systemic dose given to mice and corresponded to the mid-dose (6.0×10¹² vg Total Dose) given to mice.

Baseline chemistries and immunological studies including enzyme-linked immunosorbent spot assay (ELISpot) analysis were carried out to measure T cells against AAVrh.74 capsid and micro-dystrophin as well as anti-AAV antibody titers. Three peptide pools were used for the AAVrh.74 capsid protein (Genemed Synthesis, San Antonio, TX) containing 34-36 peptides, each 18 amino acids long and overlapping by 11 residues. Four peptide pools encompassing the micro-dystrophin.FLAG protein (Genemed Synthesis) each 18 amino acids long and overlapping by 11 residues. Concanavalin A (ConA) (Sigma, 1µg/mL) served as a positive control and 0.25% dimethylsulfoxide (DMSO) as a negative control. These studies were repeated every two weeks for the entire study. At 3 months following treatment, animals were euthanized to obtain a full tissue necropsy. Immunological assays did not show any unexpected responses to the capsid or transgene by ELISpot (Figure 12A) and no unexpected antibody responses to the AAVrh74 capsid by ELISA (Figure 12B).

In addition full complete blood count and chemistry panels showed slight elevation of liver enzymes which were normalized back to baseline with no intervention or treatment necessary, as shown in Table 3 below.

**Table 3**

| ***Blood Chemistry*** | | | | | | | |
|---|---|---|---|---|---|---|---|
| **13-176** | Baseline | 24 hours | 2 week | 4 week | 6 week | 8 week | 12 week |
| **Total Protein** (6.4-7 mg/dL) | 7 | 7 | 6.7 | 6.6 | 6.6 | 6.6 | 6.7 |
| **Billirubin,** Total (0.15-0.23 mg/dL) | 0.2 | 0.4 | 0.3 | 0.2 | 0.3 | 0.4 | 0.4 |
| **ALT** (31-50U/L) | 39 | 38 | 75 | 104 | 182 | 172 | 65 |
| **AST** (19-38 U/L) | 35 | 63 | 50 | 92 | 98 | 121 | 64 |
| **Alkaline Phosphatease** (504-821 U/L) | 417 | 396 | 332 | 383 | 598 | 608 | 578 |
| **GGT** | 77 | 77 | 120 | 106 | 131 | 156 | 134 |
| **CK** | 109 | 504 | 164 | 183 | 137 | 126 | 123 |

There were no other unexpected chemistry values throughout the duration of the study. Finally, a full analysis of all skeletal muscles demonstrated widespread expression in muscle fibers through immunofluorescence staining with a FLAG specific antibody and western blot detection using a mouse monoclonal antibody to dystrophin (Figure 14A, B).

The data together demonstrates that systemic delivery of AAVrh74.MHCK7.micro-dystrophin.FLAG established safety and efficacy with widespread expression across all skeletal muscles in a non-human primate.

### Example 8

### Pre-Clinical Study to Demonstrate Efficacy

A pre-clinical study was carried out to demonstrate efficacy of systemic delivery of rAAVrh74.MHCK7.micro-dystrophin in treating skeletal and cardiac muscle deficits in mdx mice. The AAVrh74 vector containing a codon optimized human micro dystrophin transgene driven by a muscle and cardiac specific promoter, MHCK7 as described in Example 1 was used for this study.

Systemic injections of the rAAVrh74.MHCK7.micro-dystrophin via the tail vein in mdx (dystrophin null) mice were used for a dose response study. The results of this study demonstrated that systemic injections in mdx mice was effective in normalizing histologic and functional outcomes measured in limb and diaphragm in a dose dependent manner. Additionally, no significant vector-associated toxicity was reported following formal histopathology review by a board-certified veterinary pathologist.

The vector for this study was produced by the Nationwide Children's Hospital Viral Vector Core utilizing a triple-transfection method of HEK293 cells, under research grade conditions. Characterization of the vector following production included titer determination by qPCR with a supercoiled standard, endotoxin level determination (EU/mL) and a sterility assessment. The produced vector was analyzed by SDS-PAGE to verify banding pattern consistency with expected rAAV. The vector was produced using plasmid containing the microdystrophin construct, a muscle specific MHCK7 promoter to drive expression, a consensus Kozak sequence (CCACC), an SV40 chimeric intron, synthetic polyadenylation site (53 bp) (Figure 1). The microdystrophin expression cassette was cloned between AAV2 ITRs packaged into an AAVrh74 vector for enhanced transduction of skeletal and cardiac tissue.

Potency determination of the rAAVrh74.MHCK7.micro-dystrophin test article was achieved by performing intramuscular injections of the vector into mdx mice. Wild type mice serve as a positive control and injection of sterile lactated ringers into mdx mice serve as a negative control.

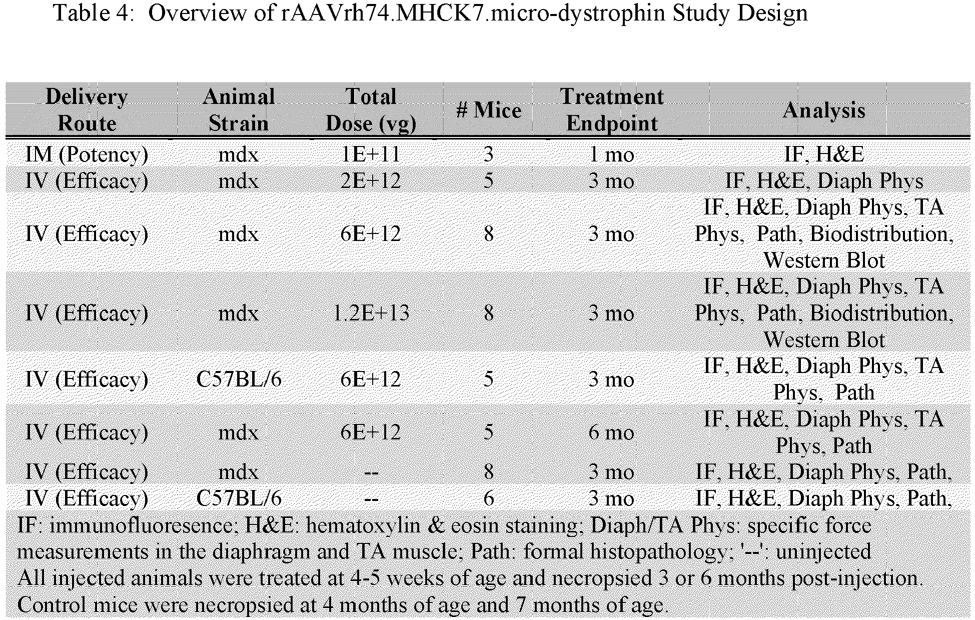

The animals indicated in Table 4 were dosed at the age indicated (4-5 weeks of age) with a tail vein injection for systemic delivery. To perform accurate dosing with an intramuscular injection, animals were briefly anesthetized by isoflurane inhalation. Doses were administered by direct injection into the tibialis anterior muscle of the lower hind limb. No anesthesia was required for accurate dosing with systemic delivery. Doses were administered by the vasculature through the tail vein. Care was taken to accurately deposit the entire vector dose into the vessel. After the dosing was performed, animals were placed on a heating pad until spontaneous movement was regained, and then returned to the cage. Observations of each animal were performed weekly for the whole duration of the study.

At the appropriate age as listed in Table 4, mice were overdosed with Ketamine/Xylazine mixture (200mg/kg/20mg/kg). Blood was collected via heart puncture and whole blood was sent for complete blood count (CBC) analysis and serum was stored at -80°C till serum chemistries were analyzed by Charles Rivers Laboratory. Tissues were then collected and sent for analysis by an independent veterinary histopathologist and in house.

Intramuscular delivery of rAAVrh74.MHCK7.micro-dystrophin to dystrophin null mice at 1×10¹¹ vg total dose resulted in ~70% expression of dystrophin in the injected TA muscles. Immunofluorescence imaging of the vector dosed mouse confirmed expression of the micro-dystrophin gene.

### Restoration of Dystrophin Expression Following Systemic Treatment with rAAVrh74.MHCK7.micro-dystrophin

Efficacy determination of the rAAVrh74.MHCK7.micro-dystrophin test article was achieved by performing systemic injections in mdx mice (*genotype: C57BL*/*10ScSn-Dmd^{mdx}*/*J*) using dose escalation at low, mid and high dose (2.0×10¹² vg Total Dose; 6.0×10¹² vg Total Dose; 1.2×10¹³ vg Total Dose) to assess transgene expression and efficacy of the vector when delivered systemically at the time points of 3 and 6 months post injection. Mice were injected at 4-5 weeks of age and a full necropsy was performed at both 3 and 6 months post-injection. Based on mean animal weights per group, these doses equal: 8×10¹³ vg/kg, 2×10¹⁴ vg/kg and 6×10¹⁴ vg/kg. Injection of an equal volume of lactated ringers served as a negative control. Injection of an equal volume of lactated ringers into C57BL/6 mice served as a positive control. Safety was determined by performing systemic injections in WT mice at a dose of 6.0 ×10¹² vg Total Dose (denoted as WT TX-mid Dose group). Immunofluorescence stain of skeletal muscles tibialis anterior (TA), gastrocnemius (GAS), quadriceps (QUAD), gluteus (GLUT), psoas, tricep (TRI), diaphragm (DIA), and heart was carried out to determine restoration of dystrophin and to ensure efficacy of viral vector of rAAVrh74.MHCK7.micro-dystrophin.

The skeletal muscles (TA, QUAD, GLUT, TRI) were extracted, along with the heart and diaphragm, for analysis. Organs were also removed for toxicology and biodistribution studies. Micro-dystrophin transgene expression remained high following 3-6 months treatment. This was accompanied by improved muscle histopathology and improved function with no adverse effects in off target organs.

### Reversal of Dystrophic Phenotype in rAAVrh74.MHCK7.micro-dystrophin Systemically Treated mdx Mice

Hematoxylin & Eosin (H&E) stain of skeletal muscles, diaphragm and heart was carried out to determine reversal and improvement of dystrophic pathology following systemic injection of rAAVrh74.MHCK7.micro-dystrophin at 2×10¹² vg total dose (Low Dose; n=1), 6×10¹² vg total dose (Mid Dose; n=8), and 1.2×10¹³ vg total dose (High Dose; n=8) for each dose with euthanasia 12 weeks post-injection. At 24 weeks post-injection, a second cohort of animals treated with mid dose (6×10¹² vg total dose) were evaluated for reversal and improvement of dystrophin pathology (n=5).

Immunofluorescence staining for the human micro-dystrophin protein was used to determine micro-dystrophin transgene expression in both left and right sides of six skeletal muscles (TA, GAS, QUAD, GLUT, psoas, TRI), as well as the diaphragm and the heart in all dystrophin null mice injected with the micro-dystrophin vector. This was carried out to determine restoration of dystrophin and to ensure efficacy of viral vector of rAAVrh74.MHCK7.micro-dystrophin at 2×10¹² vg total dose (Low Dose; n=2), 6×10¹² vg total dose (Mid Dose; n=8), and 1.2×10¹³ vg total dose (High Dose; n=8) for each dose with euthanasia 12 weeks post-injection.

In order to evaluate expression and transduction efficiency, images from all three dosing cohorts and both left and right sides of each muscle were utilized for quantification. Four 20X images were taken of each muscle and the percent of micro-dystrophin positive fibers was determined for each image resulting in the average percent transduction for each muscle. Figures 14 and 15 present representative images from treated mice from the mid dose (6×10¹²vg; 2×10¹⁴vg/kg) and the high dose (1.2×10¹³ vg; 6×10¹⁴vg/kg). Dystrophin null mice that were injected with lactated ringers and age matched were included for negative control and wild-type mice injected with lactated ringers were included for positive controls. The heart demonstrated ≥75% in all animals analyzed.

The muscles from untreated animals exhibited widespread myopathy including fatty infiltration, central nucleation, fibrosis and focal areas of necrosis. H&E staining in Figure 16 and Figure 17 illustrates this dystrophic phenotype in dystrophin null mice when compared to normal WT mice and the improvement of muscle pathology following treatment at either the mid dose (6×10¹² vg; 2×10¹⁴ vg/kg) or the high dose (1.2×10¹³ vg; 6×10¹⁴ vg/kg). Quantification of histological parameters showed a reduction in central nucleation (Figure 18) and a normalization of average fiber diameters (Figure 16 and 17) in treated mice in all muscles in a dose dependent manner. Sirius Red staining demonstrated a reduction in collagen deposition in the diaphragm in both the mid and high dose cohorts compared to untreated (mdx LR) cohorts (Figure 19).

### Functional Assessment of Systemic Treatment with rAAVrh74.MHCK7.micro-dystrophin

To determine whether micro-dystrophin gene transfer provided a functional strength benefit to diseased muscle, the functional properties of both the diaphragm and the tibialis anterior from mdx mice, WT mice, and vector dosed mice at three dose levels were assessed. The dose escalation included low dose (8×10¹³ vg/kg), mid dose (2×10¹⁴ vg/kg), and high dose (6×10¹⁴ vg/kg). Functional assessment of systemic treatment with rAAVrh74.MHCK7.micro-dystrophin using ex-vivo assessment of specific force and decrease in force output following eccentric contractions in the TA was utilized 24 weeks post-injection in animals systemically injected with rAAVrh74.MHCK7.micro-dystrophin at 6×10¹² vg total dose (Mid Dose). Additionally, specific force output in the diaphragm was assessed in the same animals.

As outlined in the previous figures, histopathology exhibited a more normalized environment with improvements in central nucleation, collagen deposition, and fiber size in the mid and high doses. Tail vein delivery of rAAVrh74.MHCK7.micro-dystrophin led to a stepwise improvement in specific force output in the diaphragm (176.9 mN/mm² in the mid dose group versus 227.78 mN/mm² in the high dose group). Additionally, the long-term treated cohort represents mice 6 months post injection (mid dose 2×10¹⁴ vg/kg) and there was no deviation in diaphragm force output long-term (176.9 mN/mm² vs 194.9 mN/mm²) (Figure 20).

Furthermore, functional deficits in tibialis anterior muscle in mdx mice were observed compared to WT mice. Mdx mice demonstrated 50% decrease in force output compared to WT mice (171.3 mN/mm² vs. 291.65 mN/mm²) and greater loss of force following eccentric contractions (32% loss in mdx; 5% loss in WT). Systemic delivery of the mid dose level of rAAVrh74.MHCK7.micro-dystrophin resulted in 65.5% dystrophin in the tibialis anterior muscle and restoration of specific force output which improved to 235.4 mN/mm2 and protected the muscle from repeated eccentric contraction damage with only a 25% decrease in force (Figure 21). The WT Mid Dose group represents a wild-type treated cohort in order to demonstrate absence of toxicity and maintenance of functional outcome measures after vector treatment.

### Summary

After the initial demonstration of biopotency by intramuscular injection, comparable or increased restoration of micro-dystrophin was achieved with vascular delivery while transducing skeletal muscles, diaphragm and the heart. The efficacy demonstrated reversal of dystrophic features in a dose dependent manner by reduction of inflammation, fewer degenerating fibers, and improved functional recovery by protecting against eccentric contractions in the tibialis anterior and diaphragm. The functional benefits of the vector include a stepwise improvement to wild-type levels in force generation of the diaphragm and the TA.

### Example 9

### Toxicology and Biodistribution of Systemic Treatment with rAAVrh74.MHCK7.micro-dystrophin

Organs and tissues from mdx mice given systemic injection of rAAVrh74.MHCK7.micro-dystrophin were collected for real-time quantitative PCR to detect specific sequences of vector DNA. Protein extracted from all collected organs and tissues were run on Western blot to detect micro-dystrophin in off-target organs.

Test article was given at three dose levels: low (2 × 10¹² vg; 8 × 10¹³ vg/kg), mid (6 × 10¹² vg; 8 × 10¹⁴vg/kg) and high dose (1.2 × 10¹³ vg; 6 × 10¹⁴vg/kg) by intravenous route at 4-5 weeks of age. To assess the safety of the vector, H&E staining was performed on cryosections of muscle tissue and all major organs harvested from the same cohorts of mice previously described. Also included were organs and muscles from C57BL6 WT mice treated systemically with the vector at the mid dose. Lactated ringers treated mdx and WT mice were also included for histopathology analysis. These sections were formally reviewed for toxicity by a third-party, board-certified, veterinary pathologist and no adverse effects were detected in any sample from any of the mice; results are summarized below.

Group details and study design are shown in 4 below.

**Table 5: rAAVrh74.MHCK7.micro-dystrophin Safety Study Design**

| **Delivery Route** | **Animal Strain** | **Total Dose (vg)** | **No. of Mice** | **Treatment Endpoint** | **Pathology Report Number** |
|---|---|---|---|---|---|
| IV | mdx | 2×10¹² | 5 | 3 mo | AAVrh74-mdx-MOUSE-001.1 |
| IV | mdx | 6×10¹² | 7 | 3 mo | AAVrh74-mdx-MOUSE-001.1/001.2 |
| IV | mdx | 1.2×10¹³ | 8 | 3 mo | AAVrh74-mdx-MOUSE-001.2 |
| IV | C57BL/6 | 6×10¹² | 5 | 3 mo | AAVrh74-mdx-MOUSE-001.2 |
| IV | mdx | 6×10¹² | 5 | 6 mo | AAVrh74-mdx-MOUSE-001.3 |
| IV | mdx | -- | 8 | 3 mo | AAVrh74-mdx-MOUSE-001.2 |
| IV | C57BL/6 | -- | 6 | 3 mo | AAVrh74-mdx-MOUSE-001.2 |

### Histopathological Review of Vector Transduced Tissue

IV injection of rAAVrh74.MHCK7.micro-dystrophin did not elicit any microscopic changes in myofibers of any skeletal muscles examined. In addition, no treatment-related lesions were seen in any of the tissues evaluated histologically. Any changes noted were seen in both treated and control mice and were considered incidental findings. Taken together, these data indicate that this test article was well tolerated by the test subjects. Furthermore, relative to reference specimens from age-matched, untreated mdx mice, administration of rAAVrh74.MHCK7.micro-dystrophin decreased myofiber atrophy in treated mdx mice, thus showing that the test article can ameliorate the degree of myopathy associated with deficiencies of mdx.

In addition to review of diseased mdx mice systemically treated with vector, rAAVrh74.MHCK7.micro-dystrophinwas delivered systemically to five C57BL/6 WT mice at a dose identical to the minimally efficacious dose (MED) established in the studies above in mdx mice, 6×10¹² vg total dose (2×10¹⁴ vg/kg). This allowed for the study of intravenous delivery of the test article in healthy WT mice to determine more definitively if any adverse effects result solely due to the treatment. Here again a variety of skeletal muscles including the diaphragm, along with heart, and five other organs were harvested and H&E sections of each tissue were formally reviewed by an independent veterinary pathologist.

### Vector Genome Biodistribution

The presence of test article-specific DNA sequences was examined using a real time, quantitative PCR assay (qPCR). Biodistribution analysis was performed on tissue samples collected from three vector dosed mdx animals per dose level. A positive signal was anything equal to or greater than 100 single-stranded DNA copies/µg genomic DNA detected. Tissues were harvested at necropsy and vector specific primer probe sets specific for sequences of the MHCK7 promoter were utilized. Figure 22 and Table 6 below depicts the vector genome copies detected in each tissue sample from rAAVrh74.MHCK7.micro-dystrophin injected mice.

**Table 6: Vector Genome copy numbers in organs and muscles from three vector dosed mdx mice per dose level. Values are shown in vg/µg genomic DNA.**

| **Tissue** | **2.00E+12** | **6.00E+12** | **1.20E+13** |
|---|---|---|---|
| | **(average vg copies/ug)** | **(average vg copies/ug)** | **(average vg copies/ug)** |
| Hrt | 2.84E+04 | 7.65E+05 | 5.35E+06 |
| Lng | 3.14E+04 | 2.52E+05 | 1.49E+06 |
| Liv | 4.36E+04 | 1.11E+07 | 1.80E+07 |
| Kid | 1.96E+04 | 3.27E+05 | 1.06E+06 |
| Spl | 5.69E+04 | 5.27E+05 | 5.78E+05 |
| Gon | 5.74E+04 | 3.68E+04 | 3.50E+05 |
| Dia | 2.22E+04 | 3.55E+05 | 2.32E+06 |
| Pso | 1.28E+05 | 1.60E+05 | 1.57E+06 |
| Tri | 1.60E+05 | 5.45E+05 | 2.50E+06 |
| Qd | 2.66E+06 | 6.57E+05 | 2.29E+06 |
| Gas | 1.69E+05 | 5.80E+05 | 2.93E+06 |
| TA | 5.86E+05 | 1.25E+05 | 1.32E+06 |

rAAVrh74.MHCK7.micro-dystrophin transcript was detected at varying levels in all collected tissues. As expected, the highest levels were seen in skeletal muscle and the heart. The lowest levels were detected in gonad, lung, kidney, and spleen. These data indicate that the test article was efficiently delivered into all investigated tissues of vector dosed mice.

As the qPCR results above indicate, intravenous delivery of rAAVrh74.MHCK7.micro-dystrophin resulted in distribution of vector transcript to varying levels in most tissues, with the highest levels occurring in the liver, heart, and quadriceps muscle (mid dose) and the liver, heart and gastrocnemius muscle (high dose). Therefore, the objective of this portion of the study was to determine the protein expression of the human micro-dystrophin transgene in these tissues to ensure the functionality of the muscle specific MHCK7 promoter. Western blotting was used to detect micro-dystrophin expression in the tissue samples.

Protein expression and vector biodistribution were also assessed using qPCR and western blotting (Figure 23), and these data indicate normal levels of vector in off-site organs and minimal detection of micro-dystrophin protein in the high dose treated livers. These results were correlated with no toxicity as determined by the pathologist in the liver. Additionally, serum chemistries were analyzed by an independent CRO (Charles River Laboratories) which indicate normal values across all chemistries analyzed. There were three abnormal values in the liver enzyme AST, 2 of which were demonstrated in the mdx-LR group and 1 of which in the mid-dose group (Figure 23). A subset of animals underwent creatine kinase analysis (CK), however, samples were analyzed pre and post physiology evaluation. Analysis of serum corroborates the lack of toxicity after test article delivery.

Micro-dystrophin protein expression was observed in varying amounts in all skeletal muscle samples as well as heart samples (Figure 24). However, there was minimal protein detected in the high dosed cohorts in the liver. This is believed to be a benign result and it might be that the presence in the liver is due to expression in smooth muscle of the liver. Importantly, there were no adverse histopathologic effects denoted by the independent pathologist report in the liver.

### Summary

Histopathology review concluded that the mdx-LR cohort exhibited widespread myopathy affecting all seven skeletal muscles evaluated as well as the right ventricular wall of the heart. The principal findings of the histopathology review included pronounced and widespread myofiber atrophy (30-75% of normal myofiber size), minimal to mild mononuclear cell inflammations, increased interstitial space, and increased cytoplasmic mineral deposits. The diaphragm exhibited the most marked changes in mononuclear cell infiltration and myofiber atrophy. The heart exhibited a few small foci of minimal mononuclear cell accumulation in the ventricular myocardium. Vector dosed cohorts had substantially reduced myopathy in all skeletal tissues and the heart. The reductions in histopathologic findings were in a dose dependent manner with the high dose group having substantially less degeneration and inflammation. There were no adverse effects due to vector treatment with rAAVrh74.MHCK7.micro-dystrophin as was documented in the WT treated cohort and the vector dosed mdx cohorts. There were incidental findings in the liver and lung of mdx and WT mice, regardless of treatment, in which the mice exhibited mild vacuolation of hepatocyte cytoplasm. Therefore, the test article was safe, efficacious and the protective effect was dose-dependent.

### REFERENCES

1. Hoffman, E.P., Brown, R.H., Jr. & Kunkel, L.M. Dystrophin: the protein product of the Duchenne muscular dystrophy locus. Cell 51, 919-928 (1987).
2. Straub, V. & Campbell, K.P. Muscular dystrophies and the dystrophin-glycoprotein complex. Curr Opin Neurol 10, 168-175 (1997).
3. Sacco, A., et al. Short telomeres and stem cell exhaustion model Duchenne muscular dystrophy in mdx/mTR mice. Cell 143, 1059-1071 (2010).
4. Wallace, G.Q. & McNally, E.M. Mechanisms of muscle degeneration, regeneration, and repair in the muscular dystrophies. Annu Rev Physiol 71, 37-57 (2009).
5. Zhou, L. & Lu, H. Targeting fibrosis in Duchenne muscular dystrophy. J Neuropathol Exp Neurol 69, 771-776 (2010).
6. Desguerre, I., et al. Endomysial fibrosis in Duchenne muscular dystrophy: a marker of poor outcome associated with macrophage alternative activation. J Neuropathol Exp Neurol 68, 762-773 (2009).
7. DiPrimio, N., McPhee, S.W. & Samulski, R.J. Adeno-associated virus for the treatment of muscle diseases: toward clinical trials. Curr Opin Mol Ther 12, 553-560 (2010).
8. Mendell, J.R., et al. Sustained alpha-sarcoglycan gene expression after gene transfer in limb-girdle muscular dystrophy, type 2D. Ann Neurol 68, 629-638 (2010).
9. Mendell, J.R., et al. Limb-girdle muscular dystrophy type 2D gene therapy restores alpha-sarcoglycan and associated proteins. Ann Neurol 66, 290-297 (2009).
10. Mendell, J.R., et al. A phase 1/2a follistatin gene therapy trial for becker muscular dystrophy. Molecular therapy : the journal of the American Society of Gene Therapy 23, 192-201 (2015).
11. Carnwath, J.W. & Shotton, D.M. Muscular dystrophy in the mdx mouse: histopathology of the soleus and extensor digitorum longus muscles. J Neurol Sci 80, 39-54 (1987).
12. Coulton, G.R., Morgan, J.E., Partridge, T.A. & Sloper, J.C. The mdx mouse skeletal muscle myopathy: I. A histological, morphometric and biochemical investigation. Neuropathol Appl Neurobiol 14, 53-70 (1988).
13. Cullen, M.J. & Jaros, E. Ultrastructure of the skeletal muscle in the X chromosome-linked dystrophic (mdx) mouse. Comparison with Duchenne muscular dystrophy. Acta Neuropathol 77, 69-81 (1988).
14. Dupont-Versteegden, E.E. & McCarter, R.J. Differential expression of muscular dystrophy in diaphragm versus hindlimb muscles of mdx mice. Muscle Nerve 15, 1105-1110 (1992).
15. Stedman, H.H., et al. The mdx mouse diaphragm reproduces the degenerative changes of Duchenne muscular dystrophy. Nature 352, 536-539 (1991).
16. Deconinck, A.E., et al. Utrophin-dystrophin-deficient mice as a model for Duchenne muscular dystrophy. Cell 90, 717-727 (1997).
17. Grady, R.M., et al. Skeletal and cardiac myopathies in mice lacking utrophin and dystrophin: a model for Duchenne muscular dystrophy. Cell 90, 729-738 (1997).
18. Love, D.R., et al. An autosomal transcript in skeletal muscle with homology to dystrophin. Nature 339, 55-58 (1989).
19. Tinsley, J.M., et al. Primary structure of dystrophin-related protein. Nature 360, 591-593 (1992).
20. Tinsley, J., et al. Expression of full-length utrophin prevents muscular dystrophy in mdx mice. Nat Med 4, 1441-1444 (1998).
21. Squire, S., et al. Prevention of pathology in mdx mice by expression of utrophin: analysis using an inducible transgenic expression system. Hum Mol Genet 11, 3333-3344 (2002).
22. Rafael, J.A., Tinsley, J.M., Potter, A.C., Deconinck, A.E. & Davies, K.E. Skeletal muscle-specific expression of a utrophin transgene rescues utrophin-dystrophin deficient mice. Nat Genet 19, 79-82 (1998).
23. Zhou, L., et al. Haploinsufficiency of utrophin gene worsens skeletal muscle inflammation and fibrosis in mdx mice. J Neurol Sci 264, 106-111 (2008).
24. Gutpell, K.M., Hrinivich, W.T. & Hoffman, L.M. Skeletal Muscle Fibrosis in the mdx/utrn+/- Mouse Validates Its Suitability as a Murine Model of Duchenne Muscular Dystrophy. PloS one 10, e0117306 (2015).
25. Rodino-Klapac, L.R., et al. Micro-dystrophin and follistatin co-delivery restores muscle function in aged DMD model. Human molecular genetics 22, 4929-4937 (2013).
26. Nevo, Y., et al. The Ras antagonist, farnesylthiosalicylic acid (FTS), decreases fibrosis and improves muscle strength in dy/dy mouse model of muscular dystrophy. PloS one 6, e18049 (2011).
27. Rodino-Klapac, L.R., et al. A translational approach for limb vascular delivery of the micro-dystrophin gene without high volume or high pressure for treatment of Duchenne muscular dystrophy. J Transl Med 5, 45 (2007).
28. Mulieri, L.A., Hasenfuss, G., Ittleman, F., Blanchard, E.M. & Alpert, N.R. Protection of human left ventricular myocardium from cutting injury with 2,3-butanedione monoxime. Circ Res 65, 1441-1449 (1989).
29. Rodino-Klapac, L.R., et al. Persistent expression of FLAG-tagged micro dystrophin in nonhuman primates following intramuscular and vascular delivery. Molecular therapy : the journal of the American Society of Gene Therapy 18, 109-117 (2010).
30. Grose, W.E., et al. Homologous recombination mediates functional recovery of dysferlin deficiency following AAV5 gene transfer. PIoS one 7, e39233 (2012).
31. Liu, M., et al. Adeno-associated virus-mediated microdystrophin expression protects young mdx muscle from contraction-induced injury. Mol Ther 11, 245-256 (2005).
32. Harper, S.Q., et al. Modular flexibility of dystrophin: implications for gene therapy of Duchenne muscular dystrophy. Nature medicine 8, 253-261 (2002).
33. Rodino-Klapac, L.R., et al. Persistent expression of FLAG-tagged micro dystrophin in nonhuman primates following intramuscular and vascular delivery. Mol Ther 18, 109-117 (2010).
34. Salva, M.Z., et al. Design of tissue-specific regulatory cassettes for high-level rAAV-mediated expression in skeletal and cardiac muscle. Mol Ther 15, 320-329 (2007).
35. Sondergaard, P.C., et al. AAV.Dysferlin Overlap Vectors Restore Function in Dysferlinopathy Animal Models. Annals of clinical and translational neurology 2, 256-270 (2015).
36. De, B.P., et al. High levels of persistent expression of alpha1-antitrypsin mediated by the nonhuman primate serotype rh. 10 adeno-associated virus despite preexisting immunity to common human adeno-associated viruses. Mol Ther 13, 67-76 (2006).
37. Rodino-Klapac, L.R., et al. A translational approach for limb vascular delivery of the micro-dystrophin gene without high volume or high pressure for treatment of Duchenne muscular dystrophy. Journal of translational medicine 5, 45 (2007).
38. Bulfield et al., X chromosome-linked muscular dystrophy (mdx) in the mouse. Proc Natl Acad Sci USA. 1984; 81(4): 1189-1192.
39. Sicinski et al., The molecular basis of muscular dystrophy in the mdx mouse: a point mutation. Science. 1989 30;244(4912):1578-80

### SEQUENCE LISTING

<110> RESEARCH INSTITUTE AT NATIONWIDE CHILDREN'S HOSPITAL
<120> ADENO-ASSOCIATED VIRUS VECTOR DELIVERY OF MUSCLE SPECIFIC MICRO-DYSTROPHIN TO TREAT MUSCULAR DYSTROPHY
<130> 28335/51475A PCT
<150> US 62/473,148
   <151> 2017-03-17
<160> 5
<170> PatentIn version 3.5
<210> 1
   <211> 3579
   <212> DNA
   <213> Homo sapiens
<400> 1
   atgctgtggt gggaggaggt ggaggattgt tatgaaaggg aggacgtgca gaagaagact      60
   tttaccaagt gggtgaacgc tcagttcagc aaatttggga agcagcacat cgagaatctg     120
   ttttccgacc tgcaggatgg gagacggctg ctggatctgc tggaaggact gactggccag     180
   aagctgccca aagagaaggg gagcactagg gtgcacgccc tgaacaacgt gaacaaagct     240
   ctgagagtgc tgcagaacaa caacgtggat ctggtgaata ttggcagtac tgatatcgtg     300
   gacgggaacc acaaactgac actgggcctg atctggaaca ttattctgca ctggcaggtg     360
   aaaaatgtga tgaagaacat catggccggg ctgcagcaga ccaattccga gaagatcctg     420
   ctgtcttggg tgcggcagag cacccgcaac tatccccagg tgaacgtgat taacttcact     480
   acatcctgga gcgacgggct ggccctgaat gctctgattc acagccacag gcctgatctg     540
   ttcgactgga atagcgtggt gtgccagcag tctgccacac agcgcctgga acatgccttc     600
   aatatcgctc ggtaccagct ggggatcgaa aaactgctgg acccagagga tgtggacact     660
   acatacccag ataaaaagtc tattctgatg tacattacta gcctgttcca ggtgctgcca     720
   cagcaggtgt ctattgaagc cattcaggag gtggaaatgc tgccccgccc ccccaaagtg     780
   actaaagagg agcattttca gctgcatcat cagatgcatt acagccagca gattaccgtg     840
   agcctggctc agggatatga gcgcaccagt agtccaaaac cacggttcaa gtcctacgct     900
   tatacccagg ctgcctacgt gacaactagc gaccctacta gatccccctt tccatcccag      960
   cacctggagg ccccagagga caagagcttt gggtccagcc tgatggaaag cgaggtgaat    1020
   ctggatcggt accagacagc cctggaggag gtgctgagct ggctgctgag tgctgaagac    1080
   acactgcagg cccagggcga aatttccaat gacgtggaag tggtgaagga tcagttccac    1140
   acacacgagg gctatatgat ggacctgaca gctcaccagg ggcgcgtggg caatatcctg    1200
   cagctgggct ctaaactgat cggcaccggg aaactgagtg aggacgagga aacagaagtg    1260
   caggagcaga tgaacctgct gaacagccgc tgggagtgtc tgagagtggc tagtatggag    1320
   aagcagtcca acctgcaccg ggtgctgatg gacctgcaga accagaaact gaaagagctg    1380
   aacgactggc tgacaaagac tgaggaacgc acaaggaaga tggaggagga gccactggga    1440
   cccgacctgg aggatctgaa gagacaggtg cagcagcata aggtgctgca ggaggatctg     1500
   gaacaggagc aggtgcgggt gaactccctg acacatatgg tggtggtggt ggacgaatct     1560
   agtggagatc acgccaccgc cgccctggag gaacagctga aggtgctggg ggaccggtgg     1620
   gccaacattt gccggtggac cgaggacagg tgggtgctgc tgcaggacat cctgctgaaa     1680
   tggcagaggc tgaccgagga gcagtgtctg tttagtgctt ggctgagcga gaaagaggac     1740
   gccgtgaaca agatccacac aaccggcttt aaggatcaga acgaaatgct gtctagcctg     1800
   cagaaactgg ctgtgctgaa ggccgatctg gagaaaaaga agcagagcat gggcaaactg     1860
   tatagcctga aacaggacct gctgagcacc ctgaagaaca agagcgtgac ccagaagaca     1920
   gaagcctggc tggataactt tgcccgctgc tgggacaacc tggtgcagaa actggagaaa     1980
   agtacagctc agatctctca ggctgtgacc acaacccagc ctagcctgac ccagacaacc     2040
   gtgatggaaa ccgtgaccac cgtgacaacc cgcgaacaga tcctggtgaa acatgcccag     2100
   gaagagctgc cacctccacc tccccagaag aagagaaccc tggagcggct gcaggagctg     2160
   caggaagcca ctgacgaact ggacctgaag ctgaggcagg ccgaagtgat taaggggtct     2220
   tggcagcctg tgggcgatct gctgattgat tccctgcagg accacctgga aaaggtgaag     2280
   gctctgagag gcgaaattgc tccactgaag gagaacgtga gtcatgtgaa cgatctggct     2340
   agacagctga caacactggg catccagctg agcccataca atctgagcac actggaggac     2400
   ctgaatacca ggtggaagct gctgcaggtg gctgtggaag accgggtgcg gcagctgcat     2460
   gaggcccatc gcgacttcgg accagccagc cagcactttc tgagcacatc cgtgcagggg     2520
   ccctgggaga gggccatttc tcccaacaag gtgccctact atattaatca cgagacccag     2580
   accacttgtt gggaccatcc caagatgaca gaactgtacc agtccctggc cgatctgaac     2640
   aacgtgaggt ttagcgctta cagaaccgct atgaagctga gacggctgca gaaggccctg     2700
   tgcctggatc tgctgtccct gtccgccgcc tgcgatgccc tggatcagca taatctgaag     2760
   cagaacgatc agccaatgga tatcctgcag atcatcaact gcctgaccac tatctacgac     2820
   aggctggagc aggagcacaa caacctggtg aacgtgcctc tgtgcgtgga tatgtgcctg     2880
   aactggctgc tgaacgtgta tgacactggg cgcaccggcc ggatcagagt gctgagtttt     2940
   aaaactggga ttatctccct gtgtaaggcc cacctggagg acaagtacag gtacctgttc     3000
   aagcaggtgg ctagtagcac tggattttgt gaccagcgcc gcctgggact gctgctgcat     3060
   gatagtatcc agattcctag acagctggga gaggtggcta gtttcggagg atctaacatc     3120
   gaacccagcg tgcgcagctg tttccagttt gccaataaca aacctgaaat cgaggctgct     3180
   ctgttcctgg attggatgcg cctggaacca cagagcatgg tgtggctgcc tgtgctgcac     3240
   agagtggctg ccgccgaaac tgccaagcac caggctaaat gcaacatctg caaggaatgt     3300
   cccattatcg gctttcgcta caggagtctg aaacatttta actacgatat ttgccagagc     3360
   tgcttctttt ccggaagagt ggccaaagga cacaagatgc actaccctat ggtggaatat     3420
   tgcaccccaa ctacatctgg cgaagatgtg cgcgattttg ccaaggtgct gaagaataag     3480
   tttcggacta agaggtactt cgccaagcac ccccgcatgg ggtatctgcc agtgcagaca     3540
   gtgctggaag gagacaatat ggagaccgat acaatgtga                            3579
<210> 2
   <211> 810
   <212> DNA
   <213> Adeno-associated virus
<400> 2
<210> 3
   <211> 8611
   <212> DNA
   <213> Adeno-associated virus
<400> 3
<210> 4
   <211> 564
   <212> DNA
   <213> Adeno-associated virus
<400> 4
   cagccactat gggtctaggc tgcccatgta aggaggcaag gcctggggac acccgagatg       60
   cctggttata attaacccag acatgtggct gctccccccc cccaacacct gctgcctgag      120
   cctcaccccc accccggtgc ctgggtctta ggctctgtac accatggagg agaagctcgc      180
   tctaaaaata accctgtccc tggtgggctg tgggggactg agggcaggct gtaacaggct      240
   tgggggccag ggcttatacg tgcctgggac tcccaaagta ttactgttcc atgttcccgg      300
   cgaagggcca gctgtccccc gccagctaga ctcagcactt agtttaggaa ccagtgagca      360
   agtcagccct tggggcagcc catacaaggc catggggctg ggcaagctgc acgcctgggt      420
   ccggggtggg cacggtgccc gggcaacgag ctgaaagctc atctgctctc aggggcccct      480
   ccctggggac agcccctcct ggctagtcac accctgtagg ctcctctata taacccaggg      540
   gcacaggggc tgcccccggg tcac                                             564
<210> 5
   <211> 8409
   <212> DNA
   <213> Adeno-associated virus
<400> 5

## Claims

1. A recombinant AAVrh.74 vector comprising an MHCK7 muscle-specific promoter/enhancer operably linked to the nucleotide sequence of SEQ ID NO: 1, wherein the recombinant AAVrh.74 vector comprises a 5' AAV2 inverted terminal repeat (ITR), the MHCK7 muscle-specific promoter/enhancer, an SV40 intron, the nucleotide sequence of SEQ ID NO: 1, a synthetic polyadenylation (PolyA) signal and a 3' AAV2 ITR.

2. The recombinant AAVrh.74 vector of claim 1, wherein the vector comprises a nucleotide sequence set forth as nucleotides 7-5042 of SEQ ID NO: 3.

3. A composition comprising the recombinant AAVrh.74 vector of claim 1 or 2, and a pharmaceutically acceptable carrier.

4. The recombinant AAVrh.74 vector of claim 1 or 2 or the composition of claim 3 for use in treatment of muscular dystrophy in a subject in need thereof.

5. The recombinant AAVrh.74 vector of claim 1 or 2 or the composition of claim 3 for use in reducing or preventing fibrosis in a subject suffering from muscular dystrophy.

6. The recombinant AAVrh.74 vector or composition for use according to claim 4 or 5, wherein the subject is suffering from Duchenne muscular dystrophy.

7. The recombinant AAVrh.74 vector or composition for use according to any one of claims 4-6, wherein the recombinant AAVrh.74 vector or composition is administered by intramuscular injection or intravenous injection.

8. The recombinant AAVrh.74 vector or composition for use according to any one of claims 4-7, wherein the recombinant AAVrh.74 vector or composition is administered systemically.

## Patentansprüche

1. Rekombinanter AAVrh.74-Vektor, der einen muskelspezifischen MHCK7-Promotor/Enhancer umfasst, der mit der Nucleotidsequenz SEQ ID Nr.: 1 funktionell verbunden ist, wobei der rekombinante AAVrh.74-Vektor ein 5'-AAV2-inverted-Terminal-Repeat (ITR), den muskelspezifischen MHCK7-Promotor/Enhancer, ein SV40-Intron, die Nucleotidsequenz SEQ ID Nr.: 1, ein synthetisches Polyadenylierungs- (PolyA-)Signal und ein 3'-AAV2-ITR umfasst.

2. Rekombinanter AAVrh.74-Vektor nach Anspruch 1, wobei der Vektor eine als Nucleotide 7-5042 mit der SEQ ID Nr.: 3 angegebene Nucleotidsequenz umfasst.

3. Zusammensetzung, die den rekombinanten AAVrh.74-Vektor nach Anspruch 1 oder 2 und einen pharmazeutisch annehmbaren Träger umfasst.

4. Rekombinanter AAVrh.74-Vektor nach Anspruch 1 oder 2 oder Zusammensetzung nach Anspruch 3 zur Verwendung bei der Behandlung von Muskeldystrophie in einem Individuum, das dessen bedarf.

5. Rekombinanter AAVrh.74-Vektor nach Anspruch 1 oder 2 oder Zusammensetzung nach Anspruch 3 zur Verwendung bei der Reduzierung oder Prävention von Fibrose in einem Individuum, das an Muskeldystrophie leidet.

6. Rekombinanter AAVrh.74-Vektor oder Zusammensetzung zur Verwendung nach Anspruch 4 oder 5, wobei das Individuum an Duchenne-Muskeldystrophie leidet.

7. Rekombinanter AAVrh.74-Vektor oder Zusammensetzung zur Verwendung nach einem der Ansprüche 4-6, wobei der rekombinante AAVrh.74-Vektor oder die Zusammensetzung durch intramuskuläre Injektion oder intravenöse Injektion verabreicht wird.

8. Rekombinanter AAVrh.74-Vektor oder Zusammensetzung zur Verwendung nach einem der Ansprüche 4-7, wobei der rekombinante AAVrh.74-Vektor oder die Zusammensetzung systemisch verabreicht wird.

## Revendications

1. Vecteur AAVrh.74 recombinant comprenant un promoteur/activateur spécifique du muscle MHCK7 lié de manière fonctionnelle à la séquence nucléotidique de la SEQ ID NO: 1, le vecteur AAVrh.74 recombinant comprenant une répétition terminale inversée (ITR) AAV2 en 5', le promoteur/activateur spécifique du muscle MHCK7, un intron SV40, la séquence nucléotidique de la SEQ ID NO : 1, un signal de polyadénylation (PolyA) synthétique et une ITR AAV2 en 3'.

2. Vecteur AAVrh.74 recombinant selon la revendication 1, le vecteur comprenant une séquence de nucléotides indiquée comme nucléotides 7-5042 de la SEQ ID NO: 3.

3. Composition comprenant le vecteur AAVrh.74 recombinant selon la revendication 1 ou 2, et un support pharmaceutiquement acceptable.

4. Vecteur AAVrh.74 recombinant selon la revendication 1 ou 2 ou composition selon la revendication 3 pour une utilisation dans le traitement d'une dystrophie musculaire chez un sujet qui en a besoin.

5. Vecteur AAVrh.74 recombinant selon la revendication 1 ou 2 ou composition selon la revendication 3 pour une utilisation dans la réduction ou la prévention d'une fibrose chez un sujet souffrant de dystrophie musculaire.

6. Vecteur AAVrh.74 recombinant ou composition pour une utilisation selon la revendication 4 ou 5, le sujet souffrant d'une dystrophie musculaire de Duchenne.

7. Vecteur AAVrh.74 recombinant ou composition pour une utilisation selon l'une quelconque des revendications 4 à 6, le vecteur AAVrh.74 recombinant ou la composition étant administré(e) par injection intramusculaire ou injection intraveineuse.

8. Vecteur AAVrh.74 recombinant ou composition pour une utilisation selon l'une quelconque des revendications 4 à 7, le vecteur AAVrh.74 recombinant ou la composition étant administré(e) de manière systémique.
